(19) Europäisches Patentamt / European Patent Office / Office européen des brevets

(11) **EP 2 744 531 B1**

(12) **EUROPÄISCHE PATENTSCHRIFT**

(45) Veröffentlichungstag und Bekanntmachung des Hinweises auf die Patenterteilung:
**21.10.2015 Patentblatt 2015/43**

(21) Anmeldenummer: **12746363.6**

(22) Anmeldetag: **15.08.2012**

(51) Int Cl.:
*A61L 31/02* (2006.01)   *A61L 31/14* (2006.01)
*C22C 23/00* (2006.01)   *C22C 23/06* (2006.01)

(86) Internationale Anmeldenummer:
**PCT/EP2012/065974**

(87) Internationale Veröffentlichungsnummer:
**WO 2013/024124 (21.02.2013 Gazette 2013/08)**

(54) **MAGNESIUMLEGIERUNG SOWIE RESORBIERBARE STENTS, WELCHE DIESE ENTHALTEN**

MAGNESIUM ALLOY AND RESORBABLE STENTS CONTAINING THE SAME

ALLIAGE DE MAGNÉSIUM ET ENDOPROTHÈSES RÉSORBABLES CONTENANT CET ALLIAGE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **15.08.2011 DE 102011110114**
**06.09.2011 US 201161573114 P**

(43) Veröffentlichungstag der Anmeldung:
**25.06.2014 Patentblatt 2014/26**

(73) Patentinhaber:
• **MeKo Laserstrahl-Materialbearbeitungen e.K.**
**31157 Sarstedt (DE)**
• **Helmholtz-Zentrum Geesthacht Zentrum für Material- und Küstenforschung GmbH**
**21502 Geesthacht (DE)**

(72) Erfinder:
• **STEKKER, Michael**
**26605 Aurich (DE)**

• **HORT, Norbert**
**21339 Lüneburg (DE)**
• **FEYERABEND, Frank**
**22529 Hamburg (DE)**

(74) Vertreter: **Arth, Hans-Lothar**
**ABK Patent Attorneys**
**Jasminweg 9**
**14052 Berlin (DE)**

(56) Entgegenhaltungen:
**WO-A1-2005/035811   WO-A1-2011/117298**
**WO-A2-2008/009825   WO-A2-2010/118193**
**US-A1- 2006 052 863**

• **DATABASE WPI Week 199631 Thomson Scientific, London, GB; AN 1996-307016 XP002683805, & JP 8 134581 A (MITSUI MINING & SMELTING CO LTD) 28. Mai 1996 (1996-05-28)**

EP 2 744 531 B1

**Beschreibung**

Die vorliegende Erfindung betrifft eine unter physiologischen Bedingungen abbaubare Magnesiumlegierung enthaltend Dysprosium, die insbesondere zur Herstellung resorbierbarer Stents geeignet ist und daraus hergestellte Stents.

Die Implantation von Gefäßstützen wie beispielsweise Stents ist heutzutage ein gängiger chirurgischer Eingriff zur Behandlung von Stenosen. Sie werden üblicherweise aus Metalllegierungen wie nicht-rostendem Edelstahl oder Nitinol hergestellt. Derartige Metallstents sind in großer Zahl bekannt und haben sich in der Praxis bewährt. Auf Grund ihrer Metallstruktur und Tragkraft sollen derartige Metallstents gewährleisten, dass die Gefäße nach Implantation offen bleiben und der Blutfluss durch die Gefäße dauerhaft gewährleistet wird.

Neuere Untersuchungen haben allerdings gezeigt, dass Gefäßstenosen nicht permanent durch eine Endoprothese, insbesondere in Form eines Stents, aufgeweitet werden müssen. Es ist vollkommen ausreichend, das Blutgefäß für einen begrenzten Zeitraum zu stützen, da das traumatisierte Gewebe des Gefäßes verheilt und die glatten Gefäßmuskelzellen regenerieren und wieder die Aufgabe übernehmen, das Blutgefäß offen zu halten und deshalb der Stent nicht länger als erforderlich im Gefäßlumen verbleiben muss.

Stents werden derzeit in zwei Grundtypen eingeteilt, dauerhafte und degradierbare bzw. resorbierbare Stents. Dauerhafte Stents sind so ausgestaltet, dass sie im Gefäß für einen unbestimmten Zeitraum verbleiben können. Resorbierbare Stents hingegen werden über einen vorbestimmten Zeitraum hinweg im Gefäß abgebaut.

Das Problem der Restenose nach Stentimplantation versucht man zurzeit zu lösen, indem man das Wachstum der glatten Muskelzellen lokal zu hemmen versucht. Dies wird z.B. mit Stents versucht, welche pharmazeutische Wirkstoffe freisetzen, die bevorzugt antiproliferativ wirken. Diese Wirkstoffe werden zumeist aus einer wirkstoffenthaltenden Beschichtung freigesetzt, welche sowohl auf dauerhaften als auch auf resorbierbaren Stents aufgebracht sein können.

Die Stützwirkung durch die Metallstruktur ist häufig nur kurzzeitig erforderlich, da sich nach Implantation des Stents das Körpergewebe erholen kann und die stützende Funktion nicht länger erforderlich ist. Vorzugsweise werden degradierbare und resorbierbare Stents erst abgebaut, wenn das traumatisierte Gewebe des Gefäßes verheilt ist und das Gefäß wieder stabilisiert, so dass der Stent nicht weiter im Gefäßlumen verbleiben muss Besonders im Falle der mit Blut in Kontakt kommenden Stents verursachen diese als körperfremdes Material die Bildung von Restenosen. Bemühungen in der Weiterentwicklung der Stents hin zu einer verbesserten Biokompatibilität des Stentmaterials, höheren Flexibilität bei geringer werdender Materialermüdung und Verkleinerung der Fremdoberfläche sollen das Risiko der Stent-induzierten Restenoserate immer weiter minimieren. Resorbierbare Stents haben hier den Vorteil, dass das körperfremde Material nicht dauerhaft im Gefäß verbleibt und die Gefahr einer Restenose somit zeitlich begrenzt wird. Ein Einsatz von resorbierbaren Stents ist auch bei Kindern von Vorteil, da hier das Gefäßwachstum nicht negativ beeinflusst wird, bzw. der Stent nach einer Weile in der das Kind gewachsen ist, nicht wieder entfernt werden muss.

Aus diesem Grund werden in neuer Zeit in zunehmendem Maße Stents aus bioresorbierbaren Materialien wie z.B. aus Polymeren wie z.B. Polyhydroxybutyrat oder aus Metallen wie Magnesium oder Eisen entwickelt und in klinischen Versuchen eingesetzt.

Die großen Rückstellkräfte der Gefäße nach einer Aufdehnung sind ein wichtiger Grund für Restenosen. Daher müssen resorbierbare Gefäßstützen aus einem Material bestehen, welches zwar gut vom Körper abgebaut werden kann, aber auch eine ausreichend hohe Rückhaltekraft aufweist, um einen erneuten Verschluss des Gefäßes zu verhindern.

Ein einmal eingesetzter Stent muss seine Größe und Form beibehalten, trotz der unterschiedlichen Kräfte, die auf ihn einwirken, wie z.B. die pulsierende Belastung durch das schlagende Herz. Darüber hinaus muss der Stent genügend Flexibilität besitzen, um auf einen Ballon gekrimpt und später im Gefäß expandiert zu werden.

Resorbierbare Polymere, welche zur Herstellung von Stents verwendet werden, weisen geringere mechanische Festigkeitswerte auf als die bislang verwendeten nicht resorbierbaren Metalllegierungen. Eine Kompensation dieses Nachteils kann durch größere Stegbreiten des Stents erfolgen. Dies erhöht jedoch die mechanische Reizung der Gefäßwandung während der Stentimplantation und damit auch die Restenosegefahr. Resorbierbare Stents aus Eisen oder einer eisenbasierten Legierung haben den Nachteil, dass die Verweildauer im Gefäß bis zum vollständigen Abbau länger als notwendig und gewünscht ist. Für resorbierbare Stents liegt der angestrebte Zeitraum der Resorption zwischen 3 und 12 Monaten, wobei die mechanische Belastbarkeit zuvor gewährleistet sein muss. Magnesium ist als Spurenelement im Körper vorhanden und daher als Basis für einen resorbierbaren Stent geeignet. Des Weiteren wurden Legierungsbestandteile aus den Metallen der Gruppe der seltenen Erden gewählt, da diese im Körper natürlich nicht vorkommen. Dies ermöglicht einen Nachweis der Degradationsprodukte im Gewebe und in den Organen.

Magnesium und Magnesiumlegierungen besitzen für eine Vielzahl von Anwendungen hervorragende mechanische und physikalische Eigenschaften. Ihr geringes Gewicht bei gleichzeitig hoher Festigkeit machen Magnesium und Magnesiumlegierungen zu geeigneten Materialien auch für Endoprothesen. Magnesium und Magnesiumlegierungen sind sehr reaktionsfreudig und daher korrosionsanfällig. Für resorbierbare Implantate sind diese Eigenschaften jedoch wünschenswert. Allerdings bestehen folgende Probleme im Stand der Technik: Obgleich grundsätzlich das angestrebte Ziel einer Resorption des implantierten Stents erreicht wird, besteht das Problem eines zeitlich nicht definierten Abbaus des Stents. Je nach Materialwahl ist der Materialabbau starken Schwankungen unterworfen, nicht kontrollierbar und im Allgemeinen zu schnell, um ein sicheres Einwachsen des Stents in die Gefäßwände zu gewährleisten. Bei zu schneller Resorption kann der resorbierbare Stent nicht in die Gefäßwand einwachsen und die Stützfunktion bis zur Regeneration des Gefäßabschnitts übernehmen. Er kann sich vielmehr ablösen bzw. Stücke des Stents können sich ablösen und im Blutfluß mitgerissen werden und lebensbedrohliche Probleme für den Patienten verursachen.

Ein bioresorbierbarer Metallstent aus Magnesium und Yttrium ist in dem europäischen Patent EP 1 419 793 B1 offenbart. Eine Magnesiumlegierung mit Yttrium, Neodym und weiteren optionalen Bestandteilen geeignet für die Herstellung von Stents wird in dem europäischen Patent EP 2 169 090 beschrieben. Diese Stents haben den Nachteil, dass sie sich zu schnell und zudem unkontrolliert auflösen. Da der Auflösungsprozess zumeist beginnt, bevor der Stent in die Gefäßwand eingewachsen ist, können sich Bruchstücke lösen, durch die Blutbahn transportiert werden und einen Herzinfarkt auslösen. Ferner hat sich herausgestellt, dass diese Stents aus einer Magnesium-Yttrium-Legierung die Ablagerung von Calciumphosphat auf der luminalen Oberfläche der Stents begünstigen und damit zu einem Wiederverschluß des Stents (In-Stent Restenose) und damit auch des Gefäßes führen, was gerade verhindert werden soll.

Die europäischen Patentanmeldungen EP 2 213 314 A1 und EP 1 842 507 A1 offenbaren ebenfalls Stents aus einer Magnesiumlegierung enthaltend Gadolinium. Um die gewünschten mechanischen Eigenschaften wie beispielsweise Festigkeit, Spannkraft oder Duktilität zu erhalten, wird Gadolinium in Mengen größer 5 Gew.-% benötigt. Bei Mengen größer 5 Gew.-% Gadolinium tritt jedoch das Problem auf, dass die Verarbeitbarkeit der Legierung zu einem Rohr geeignet für die Laserbearbeitung und die Homogenität der Legierung nicht mehr gewährleistet ist. Somit kann festgestellt werden, dass bis August 2012 keine Metalllegierung im Stand der Technik existiert, welche sich als Material für die Herstellung von bioresorbierbaren Stents eignen würde.

Aus diesem Grund besteht das Bedürfnis einen geeigneten Werkstoff für resorbierbare Stents zu entwickeln. Aufgabe der vorliegenden Erfindung ist es, ein geeignetes Material und eine daraus hergestellte Gefäßstütze bereitzustellen, welche ihre Stützfunktion mindestens so lange ausübt, bis das regenerierte Gewebe wieder selber in der Lage ist, diese Funktion zu übernehmen.

Fasst man die Aufgabe konkreter, so besteht die Aufgabe der vorliegenden Erfindung darin, eine Magnesiumlegierung bereitzustellen, deren Auflösungskinetik gegenüber den bekannten Magnesiumlegierungen verzögert bis deutlich verlangsamt ist.

Diese Aufgabe wird durch die technische Lehre der unabhängigen Ansprüche der vorliegenden Erfindung gelöst. Weitere vorteilhafte Ausgestaltungen der Erfindung ergeben sich aus den abhängigen Ansprüchen, der Beschreibung sowie den Beispielen.

Es hat sich überraschend gezeigt, dass Magnesiumlegierungen mit einem relativ hohen Gehalt an Dysprosium, der zudem bevorzugt Neodym und/oder Europium und optional Zirkonium und/oder Zink zugemischt werden, sich durch ein vorteilhaftes Korrosionsverhalten, eine gewünschte Resorptionskinetik und für die Herstellung von Stents geeigneten mechanischen Eigenschaften auszeichnen.

Die vorliegende Erfindung betrifft daher resorbierbare Stents bestehend aus Magnesiumlegierungen, die bezogen auf das Gesamtgewicht der Legierung (angegeben in Gew.-%) folgende Bestandteile enthalten:

| | | | |
|---|---|---|---|
| 64,5 Gew.-% | - | 94,79 Gew.-% | Magnesium |
| 5,0 Gew.-% | - | 25,5 Gew.-% | Dysprosium |
| 0,01 Gew.-% | - | 5,0 Gew.-% | Neodym und/oder Europium |
| 0,1 Gew.-% | - | 3,0 Gew.-% | Zink |
| 0,1 Gew.-% | - | 2,0 Gew.-% | Zirkonium |

Diese Legierung besteht aus 5,0 bis 25,5 Gew.-% Dy und 0,01 bis 5,0 Gew.-% Nd oder 0,01 bis 5,0 Gew.-% Eu oder 0,01 bis 5,0 Gew.-% Nd und Eu zusammen, 0,1 Gew.-% - 3,0 Gew.-% Zink und 0,1 Gew.-% - 2,0 Gew.-% Zirkonium, wobei der restliche Anteil bis hin zu 100 Gew.-% Mg ist. Diese Legierung kann ferner noch unvermeidbare Verunreinigungen enthalten. Bevorzugte Bereiche für die Bestandteile Dy, Nd, Eu, Zn und Zr werden weiter unten eingehend beschrieben.

Die vorliegende Erfindung betrifft zudem resorbierbare Stents aus Magnesiumlegierungen enthaltend 5,0 bis 25,5 Gew.-% Dy und 0,01 bis 5,0 Gew.-% Nd oder 0,01 bis 5,0 Gew.-% Eu oder 0,01 bis 5,0 Gew.-% Nd und Eu zusammen, 0,1 Gew.-% - 2,0 Gew.-% Zirkonium, des Weiteren umfassend 0,1 Gew.-% - 2,0 Gew.-% Zink.

Die vorliegende Erfindung betrifft zudem resorbierbare Stents aus Magnesiumlegierungen enthaltend 5,0 bis 25,5 Gew.-% Dy und 0,01 bis 5,0 Gew.-% Nd oder 0,01 bis 5,0 Gew.-% Eu oder 0,01 bis 5,0 Gew.-% Nd und Eu zusammen, 0,1 Gew.-% - 3,0, bevorzugt bis 2,0 Gew.-% Zink, des Weiteren umfassend 0,1 Gew.-% - 0,3 Gew.-% Zirkonium. Auch diese Legierungen können ferner noch unvermeidbare Verunreinigungen enthalten.

Die vorliegende Erfindung betrifft des Weiteren Magnesiumlegierungen, die bezogen auf das Gesamtgewicht der Legierung (angegeben in Gew.-%) folgende Bestandteile enthalten:

| | | | |
|---|---|---|---|
| 81,5 Gew.-% | - | 91,9 Gew.-% | Magnesium |
| 7,0 Gew.-% | - | 15,0 Gew.-% | Dysprosium |
| 0,5 Gew.-% | - | 1,5 Gew.-% | Neodym und/oder Europium |
| 0,5 Gew.-% | - | 1,5 Gew.-% | Zink |
| 0,1 Gew.-% | - | 0,5 Gew.-% | Zirkonium |

Magnesium (Mg) als Hauptbestandteil der Legierung wurde ausgewählt, da Mg biologisch abbaubar und ein notwendiger Körperbestandteil ist, der sich nicht so im Körper anreichert, dass er schädlich wirkt. Überschüssiges Magnesium wird in der Regel auf natürlichem Wege ausgeschieden.

Dysprosium bildet zusammen mit Magnesium intermetallische Ausscheidungen. Die hohe Löslichkeit von Dysprosium in Magnesium sorgt zudem dafür, dass man die bei der Herstellung von Stents erforderlichen Wärmebehandlungen erfolgreich durchführen kann, sich Ausscheidungen auflösen und gezielt erneut ausscheiden und man so Eigenschaften wie z.B. Festigkeit, Duktilität und Korrosionsverhalten in einem weiten Rahmen einstellen kann. Eine hohe Festigkeit und eine hohe Duktilität verlangsamen den biologischen Abbau der Legierung, was bei einem Stent aus einer Magnesiumlegierung gerade gewünscht ist. Die Duktilität sollte mindestens bei 15% bevorzugt mindestens bei 20%, weiter bevorzugt zwischen 20% und 25% und insbesondere bevorzugt mindestens bei 25% liegen. Wie hierin verwendet, werden die Begriffe "Duktilität" und "Bruchdehnung" synonym verwendet. Die Streckgrenze als ein Maß für Festigkeit sollte in einem Bereich von 80 MPa-180 MPa liegen.

Dysprosium steigert zudem die Festigkeit der Legierung, da es im Mischkristall gelöst ist und Ausscheidungen bilden kann. Europium bildet nur Ausscheidungen ähnlich wie Neodym. Im einzelnen Korn der hierin beschriebenen Legierungen, die sowohl Dysprosium als auch Europium und/oder Neodym enthalten, kann daher Mischkristallhärtung und Ausscheidungshärtung verbunden werden. Bei einer Wärmebehandlung kann man die Magnesium-Dysprosium-Ausscheidungen auflösen und gezielt wieder ausscheiden. Damit kann durch die hierin beschriebene Legierungszusammensetzung die Festigkeit und Duktilität in einem weiten Bereich eingestellt werden. Sobald jedoch alle Ausscheidungen auf den Korngrenzen verschwunden sind, können Körner anfangen zu wachsen (Ostwald-Reifung). Grobe Körner haben jedoch einen negativen Einfluss auf Festigkeit und Duktilität und sollen daher vermieden werden. Magnesium-Europium- oder Magnesium-Neodym-Ausscheidungen auf den Korngrenzen stabilisieren die Korngrenzen während einer Wärmebehandlung, welche bei der Herstellung von Stents immer erforderlich ist. Die Korngröße ändert sich daher aufgrund des stabilisierenden Effektes von Europium und/oder Neodym nicht. Es ist erstrebenswert ein vorliegendes, feines Korn auf jeden Fall zu stabilisieren, da nach Hall-Petch ein feinkörniges Gefüge sich positiv auf Festigkeit und Duktilität auswirkt. Diese Stabilisierung wird erfindungsgemäß bei einer Mg-Dy-Legierung durch den Zusatz von Europium und/oder Neodym erreicht.

Für die Herstellung von Stents wurde insbesondere auf die Festigkeitseigenschaften und das Korrosionsverhalten geachtet, um eine möglichst feste und korrosionsbeständige Legierung bereitzustellen.

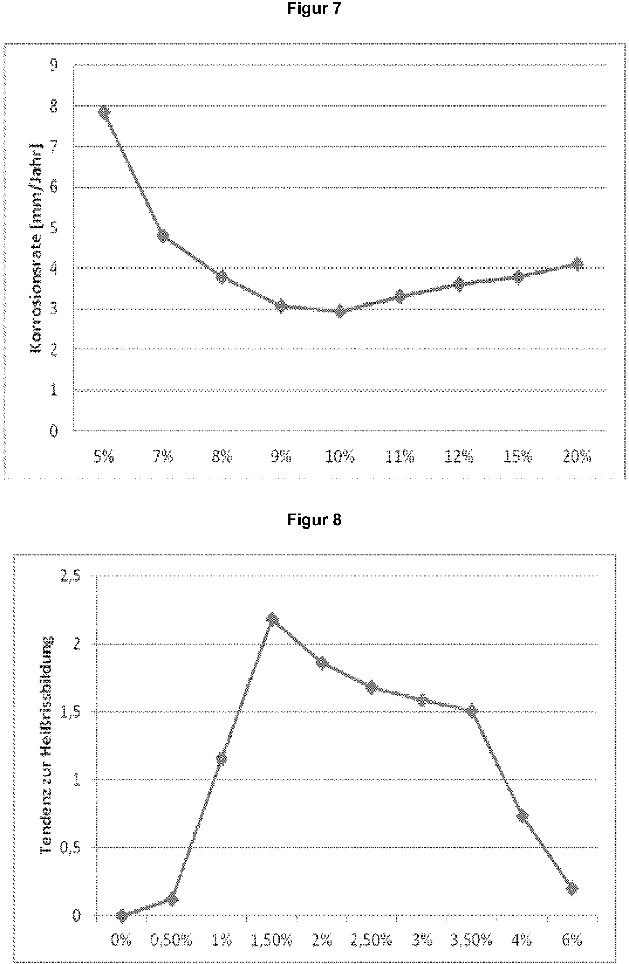

Es wurde gefunden, dass das Minimum der Korrosion einer erfindungsgemäßen Magnesiumlegierung bei einem Gehalt von 10 Gew.-% Dy liegt. Daher ist es besonders bevorzugt, wenn der Gehalt an Dysprosium in den erfindungsgemäßen Legierungen bei circa 10 Gew.-% ± 2 Gew.-% liegt. Aus Figur 7 geht hervor, dass im Bezug auf die Korrosion eine binäre Magnesiumlegierung enthaltend Dysprosium, bei einer Menge von 7 bis 15 Gew.-%, noch besser 8 - 12 Gew.-% Dysprosium ein bevorzugtes minimales Korrosionsverhalten zeigt. Die Korrosion ist die entscheidende Eigenschaft für die Abbaurate des Stents im Gefäß. Es ist wichtig, dass ein biodegradierbarer Stent nicht zu früh seine Stabilität verliert, so dass sich keine Bruchstücke ablösen und die Stabilität durch den Stent gewährleistet wird, bis diese wieder von dem Gefäß alleine aufgebracht werden kann und bis der Stent in die Gefäßwand eingewachsen ist.

Auch Neodym und Europium haben *in vitro* keine negativen Effekte auf Zellen gezeigt. Europium war hier gegenüber Neodym sogar noch etwas besser verträglich. Beide Elemente sind praktisch unlöslich in Magnesium und bilden intermetallische Phasen mit Magnesium, die, auch durch die bei der Herstellung von Stents notwendigen Wärmebehandlungen, nicht aufgelöst werden. Diese Ausscheidungen befinden sich auf den Korngrenzen und stabilisieren diese, so dass das feine Korn aus der Umformung beibehalten wird. Erfindungsgemäß hat sich gezeigt, dass dazu 1 Gew.-% Neodym oder 1 Gew.-% Europium oder 1 Gew.-% von Europium und Neodym zusammen ausreichen. Mengen an Europium und/oder Neodym größer als 1 Gew.-% beginnen die Duktilität der Legierung zu verringern, was für die Herstellung von Stents unerwünscht ist und Mengen an Europium und/oder Neodym größer als 2 Gew.-% reduzieren die Duktilität der Legierung derart, dass die notwendige minimale Duktilität von 15% nicht mehr gewährleistet war. Mengen an Eu und/oder Nd von mehr als 2 Gew.-% haben mit weiter steigendem Gewichtsanteil eine Erhöhung der Versprödung der Legierung zur Folge und eine Erniedrigung der Duktilität.

Gerade die Duktilität einer Legierung ist jedoch wesentlich für die Eignung als ein Werkstoff für die Stentherstellung. Nach der Fertigung wird der Stent auf einen Träger, zumeist einen Katheterballon gecrimpt und damit das erste Mal plastisch verformt. Vor Ort im Blutgefäß wird der Stent anschließend aufgeweitet und erneut plastisch verformt. Um diese beiden doch recht drastischen Umformvorgänge ohne Schädigung durchführen zu können, ist eine hohe Bruchdehnung und damit eine hohe Duktilität erforderlich. Gleichzeitig ist jedoch auch eine hohe Festigkeit erforderlich, um ein Reißen der Stege des Stents bei den beiden Umformvorgängen zu vermeiden und ein Zusammendrücken des Stents und damit den Verschluss des Gefäßes durch die Rückstellkräfte der Gefäßwand zu verhindern. Bei den möglichen Mechanismen zur Festigkeitssteigerung ist der Hall-Petch-Mechanismus (=Feinkornhärtung) dazu geeignet, hohe Festigkeiten zu erreichen und dabei gleichzeitig die Duktilität zu steigern. Alle Legierungselemente, die daraus resultierenden intermetallischen Phasen und auch die durch die Verformung des Stents erzeugte Kaltverformung steigern zwar die Festigkeit, verringern jedoch gleichzeitig die Duktilität. Um dem entgegen zu wirken, ist daher ein feines Korn unerlässlich.

Zink verbessert das Giessverhalten - der Magnesiumlegierung und hat eine festigkeitssteigernde Wirkung. So kann durch einen Zinkzusatz bis zu 3 Gew.-% die Schwing- und Zugfestigkeit erhöht werden. Die Zugfestigkeit sollte vorzugsweise so groß wie möglich sein und vorzugsweise über 180 MPa ($\geq$180 MPa), weiter bevorzugt über 200 MPa ($\geq$200 MPa) liegen. Allerdings steigt bei mehr als 1 Gew.-% Zn die Tendenz zur Heißrissbildung (s. Figur 8). Dadurch entstehen Mikroporen, die sich negativ auf die Zugfestigkeit und Duktilität einer Legierung auswirken. Sie wirken als innere Kerben, so dass ein Werkstoff im Zugversuch generell deutlich unterhalb der maximal erreichbaren Festigkeit bei einem Bruchteil der theoretischen Duktilität versagt. Generell zeigt sich bei mehr als 2 Gew.-% Zink nachteilige Wirkungen auf das Verarbeitungsverhalten und die mechanischen Eigenschaften der hierin beschriebenen Legierungen. Zink ist ein essentielles Element für den Menschen, welches Bestandteil vieler Enzyme ist und hat viele Funktionen. Zink hat unter anderem eine entzündungshemmende Wirkung. Dennoch kann bei hohen Dosen eine akute Vergiftung auftreten und eine längerfristige Zufuhr führt zu Störungen, insbesondere des Eisen- und Kupferhaushaltes (vgl. Guidelines for drinking-water quality, World Health Organization, 1996). Bei einem Zinkgehalt von 4 Gew.-% und mehr können daher toxische Nebenwirkungen nicht ausgeschlossen werden. Der Gehalt an Zink sollte daher unterhalb von 2,0 Gew.-%, bevorzugt unterhalb von 1,8 Gew.-%, weiter bevorzugt unterhalb von 1,6 Gew.-%, noch weiter bevorzugt unterhalb von 1,4 Gew.-% und insbesondere bevorzugt unterhalb von 1,2 Gew.-% liegen. Als untere Grenze sollten 0,1 Gew.-% Zn, vorzugsweise 0,3 Gew.-% Zn und insbesondere 0,5 Gew.-% Zn nicht unterschritten werden.

Neben Zink oder auch an Stelle von Zink kann zudem Zirkonium (Zr) in der Magnesiumlegierung enthalten sein. Zr wird erfindungsgemäß als Kornfeinungsmittel eingesetzt. Zr kann zudem Fe abbinden und damit dessen Gehalt weiter vermindern. Es wurde gefunden, dass elementares Eisen die Korrosion erhöht, was erfindungsgemäß vermieden werden sollte. Dies könnte dadurch begründet werden, dass elementares Eisen mit Mg ein galvanisches Element bildet, wobei auch andere Ursachen denkbar wären. Vorzugsweise legiert man Zr in einer Größenordnung von bis zu ca. 0,4 Gew.-% zu. Auch größere Mengen an Zr von 2 Gew.-% oder auch 3 Gew.-% führen zu einer ähnlich guten Kornfeinung, verteuern aber die Legierung deutlich und führen zudem zu einer Versprödung der Legierung, was wiederum zu einer Erniedrigung der Duktilität führt. Zr und Mg zeigen in der Mg-reichen Ecke ihres Zustandsdiagrammes eine peritektische Reaktion. Das heißt, dass sich bei der Erstarrung primär reines Zr ausscheidet. Auf Grund der hexagonalen Gitterstruktur und Gitterparameter, die dem von Mg sehr ähnlich sind, wirkt Zr als Kornfeinungsmittel. Dabei müssen die Zr-Keime jedoch erst einen Durchmesser von etwa 2 $\mu$m und größer erreichen. Zr hat jedoch eine deutlich höhere Dichte als Mg. Dadurch sinken die Zr-Partikel in einer Mg-Schmelze vergleichsweise rasch auf den Boden. Von einer eingesetzten Menge an Zr von 1 Gew.-% ist daher nur die Hälfte tatsächlich effektiv als Kornfeinungsmittel nutzbar. Der Rest scheidet sich in der Regel auf dem Tiegelboden ab. Rühren während des Erstarrens kann erfolgreich eingesetzt werden, um dieses Absetzen zu unterdrücken. Dies ist jedoch aufwendig und auch nicht unter allen Bedingungen machbar. In der Regel verliert man daher ca. die Hälfte des eingesetzten Zr. Dies ist unter anderem ein wesentlicher Kostenpunkt. Da aber mit deutlich geringeren Mengen an Zr von 0,05 Gew.-% bis 0,50 Gew.-% genau so gute Resultate hinsichtlich der Kornfeinung als mit 1 Gew.-%, 2 Gew.-% oder 3 Gew.-% erzielt wurden und zudem bei einer Menge an Zr unterhalb von 0,50 Gew.-% keine Versprödung auftritt, werden 0,05 Gew.-% bis 0,50 Gew.-% und weiter bevorzugt 0,08 Gew.-% bis 0,4 Gew.-% Zirkonium eingesetzt.

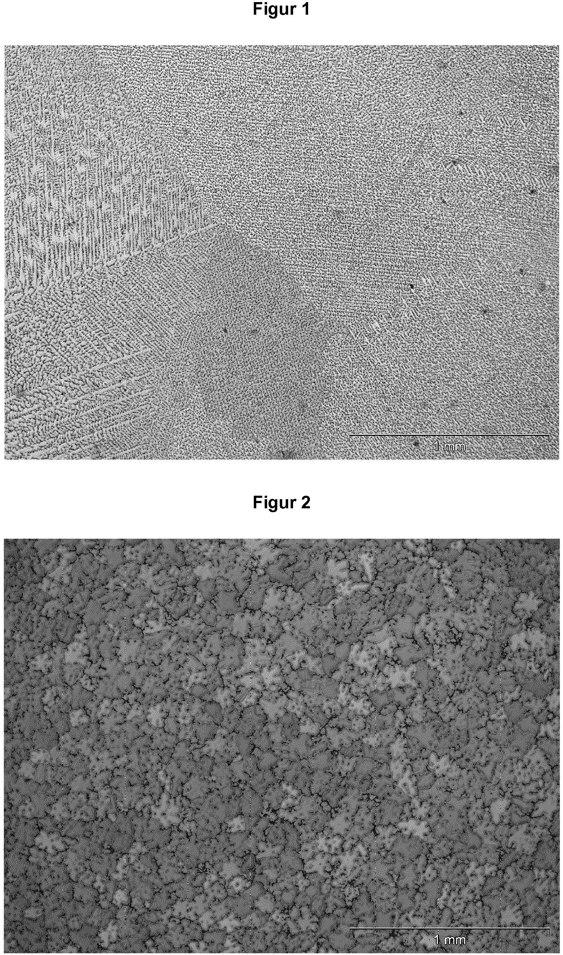

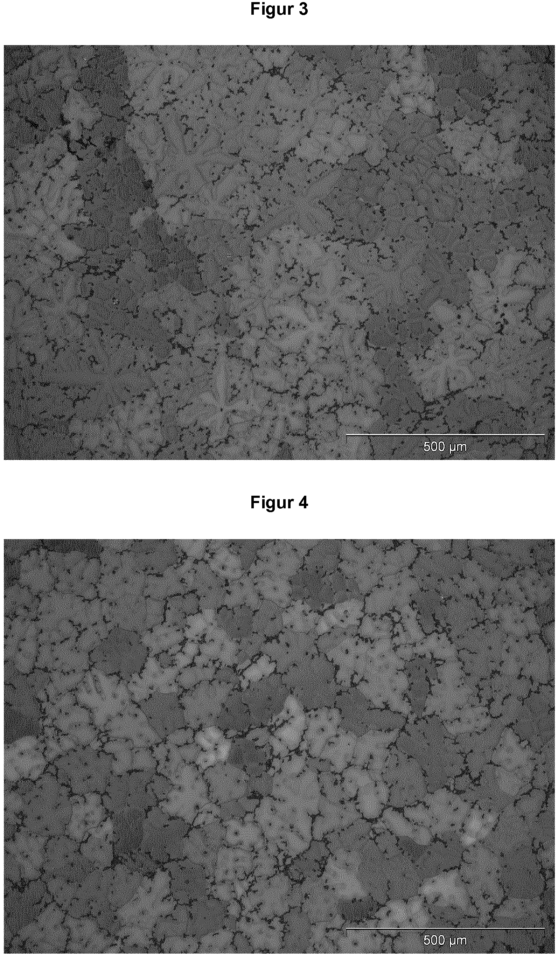

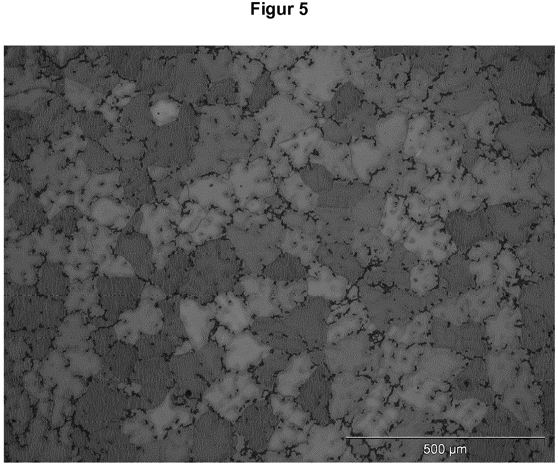

Der Einfluss von Zr wurde beispielhaft an der Magnesiumlegierung enthaltend 10 Gew.-% Dy und 1 Gew.-% Nd untersucht. Als Herstellungsverfahren wurde das Tütenguß-Verfahren eingesetzt. Bei den im Tütenguß hergestellten Werkstoffen kann man davon ausgehen, dass ein Gußteil eine homogene Gefügestruktur zeigt und dass auch die Legierungselemente homogen verteilt sind. Allerdings ist das Gefüge auch vergleichsweise grob und die Korngröße liegt im Bereich mehrerer Millimeter (Abb. 1). Die Erfinder konnten zeigen, dass die Zugabe von nur 0,6 Gew.-% Zr dagegen eine deutliche Abnahme der Korngröße bewirkt (Abb. 2). Es wurden daher drei unterschiedlich große Zr-Anteile (0,2, 0,4, 0,6 Gew.-%) und ihr Einfluss auf das sich ausbildende Gefüge untersucht. Zur Ermittlung der Korngröße wurde das Linienschnittverfahren angewandt. Überraschenderweise hat bereits ein geringer Anteil von 0,2 Gew.-% eine deutliche Kornfeinung zur Folge (Abb. 2) und die Korngröße liegt im Bereich von 102 $\mu$m. Die Zugabe von 0,4 bzw. 0,6 Gew.-% hat Korngrößen im Bereich von 68 $\mu$m bzw. 64 $\mu$m zur Folge (Abb. 4 und 5). Man kann daher schlussfolgern, dass bereits eine Zugabe von 0,2 Gew.-% Zr eine effektive Kornfeinung verursacht und dass überraschenderweise das gesamte Zr für die Kornfeinung aktiviert werden kann. Dies senkt allein die Kosten für Zr um ca. 50 %.

Es ist daher bevorzugt, wenn eine hierin beschriebene Legierung des Weiteren 0,02 - 0,80 Gew.-%, bevorzugt 0,04 - 0,60 Gew.-%, bevorzugt 0,05 - 0,55 Gew.-%, weiter bevorzugt 0,06 - 0,50 Gew.-%, noch weiter bevorzugt 0,07 - 0,45 Gew.-%, noch weiter bevorzugt 0,08 - 0,40 Gew.-%, noch weiter bevorzugt 0,09 - 0,35 Gew.-%, noch weiter bevorzugt 0,10 - 0,30 Gew.-%, noch weiter bevorzugt 0,12 - 0,28 Gew.-% und insbesondere bevorzugt 0,15 - 0,25 Gew.-% Zirkonium aufweist.

Die Korngrößen wurden gemäß dem bekannten Linienschnittverfahren bestimmt. Im Linienschnittverfahren wird die Auszählung der Korngröße im Okular, auf einer Mattscheibe oder auf Fotos vorgenommen. Die Schnittlinien können entweder gerade oder kreisförmig sein. Körner die am Ende der Geraden nur halb geschnitten sind werden hierbei als halbe Körner gezählt. Die Vergrößerung wird so gewählt, dass durch das Linienraster mindestens 50 Körner geschnitten werden. Es müssen mindestens 5 Stellen, mit einer Gesamtanzahl von mindestens 250 Schnittpunkten, auf der Probe ausgewertet werden.

Die vorliegende Erfindung betrifft zudem bevorzugt resorbierbare Stents aus Magnesiumlegierungen, die bezogen auf das Gesamtgewicht der Legierung (angegeben in Gew.-%) folgende Bestandteile enthalten:

| 80,7 Gew.-% | - | 94,7 Gew.-% | Magnesium |
|---|---|---|---|
| 5,0 Gew.-% | - | 15,0 Gew.-% | Dysprosium |
| 0,1 Gew.-% | - | 2,0 Gew.-% | Neodym |
| 0,1 Gew.-% | - | 2,0 Gew.-% | Zink |
| 0,1 Gew.-% | - | 0,3 Gew.-% | Zirkonium |

Optional kann in dieser Legierung der Anteil an Neodym durch Europium ersetzt werden, oder es können 0,1 Gew.-% - 2,0 Gew.-% Europium zusätzlich zugesetzt werden.

Es versteht sich von selbst, dass alle Bestandteile einer Legierung zusammen 100 Gew.-% ergeben müssen. Enthält obige Legierung somit 15,0 Gew.-% Dysprosium (Dy) und 2,0 Gew.-% Neodym (Nd) so kann der Magnesiumanteil nicht über 83 Gew.-% liegen. Sind neben Magnesium, Dysprosium, Neodym / Europium, Zink und Zirkonium noch andere Bestandteile anwesend, so handelt es sich dabei um die hierin beschriebenen Verunreinigungen wie beispielsweise die anderen Metalle, Metallsalze, Nichtmetalle, Kohlenstoff, Schwefel, Stickstoff, Sauerstoff, Silizium und/oder Wasserstoff.

Sofern nicht explizit aufgeführt, können die hierin offenbarten Legierungen unvermeidbare Verunreinigungen enthalten, welche im Bereich von der Nachweisgrenze oder im Bereich von 1 ppm bis zu 0,4 Gew.-%, bevorzugt bis zu 0,3 Gew.-%, weiter bevorzugt bis zu 0,2 Gew.-% und besonders bevorzugt bis zu 0,1 Gew.-% liegen. Silizium als Hauptbestandteil der Verunreinigungen kann dabei bereits 0,3 Gew.-% ausmachen. Es ist daher besonders bevorzugt, wenn die unvermeidbaren Verunreinigungen außer Silizium insgesamt weniger als 0,3 Gew.-%, bevorzugt weniger als 0,2 Gew.-%, weiter bevorzugt 0,1 Gew.-%, weiter bevorzugt 0,05 Gew.-%, weiter bevorzugt 0,01 Gew.-%, weiter bevorzugt 0,001 Gew.-%, weiter bevorzugt weniger als 500 ppm und insbesondere bevorzugt weniger als 300 ppm betragen. Die vorgenannten Prozentzahlen beziehen sich auf die Summe aller Verunreinigungen außer Silizium und nicht auf die einzelne Verunreinigung. Diese Verunreinigungen (einschließlich Si) können auch dann in einer Menge von 1 ppm bis hin zu 0,4 Gew.-% oder 0,3 Gew.-% oder 0,2 Gew.-% oder 0,1 Gew.-% in der Legierung vorhanden sein, wenn sie nicht explizit als Legierungsbestandteil aufgeführt sind und werden im Fall der Nichtnennung dem Gewichtsanteil des Bestandteils der Legierung zugerechnet, durch den sie in die Legierung gelangt sind. Bevorzugt ist jedoch, wenn die Verunreinigungen außer Silizium jeweils eine Menge von 500 ppm, bevorzugt 300 ppm, weiter bevorzugt 200 ppm und insbesondere bevorzugt 150 ppm nicht überschreiten. Silizium kann ein Hauptbestandteil der Verunreinigungen sein und bis zu 0,3 Gew.-%, bevorzugt bis zu 0,2 Gew.-% und weiter bevorzugt bis zu 0,1 Gew.-% in der Legierung vorhanden sein. Je nachdem wie das Magnesium gewonnen wird, kann das Magnesium einen unterschiedlich hohen Gehalt an Si aufweisen. Wird sehr reines Magnesium eingesetzt, so kann auch der Gehalt an Si in der Legierung unterhalb von 400 ppm, bevorzugt unterhalb von 300 ppm, weiter bevorzugt unterhalb von 250 ppm und insbesondere bevorzugt unterhalb von 200 ppm liegen.

Hierin offenbart werden ferner Magnesiumlegierungen, die bezogen auf das Gesamtgewicht der Legierung aus folgenden Bestandteilen bestehen:

| 76,0 Gew.-% | - | 95,0 Gew.-% | Magnesium |
|---|---|---|---|
| 5,0 Gew.-% | - | 25,5 Gew.-% | Dysprosium |
| 0,0 Gew.-% | - | 5,0 Gew.-% | Neodym |
| 0,0 Gew.-% | - | 5,0 Gew.-% | Europium |
| 0,0 Gew.-% | - | 3,0 Gew.-% | Zink |
| 0,0 Gew.-% | - | 1,5 Gew.-% | Zirkonium |

| 0,0 Gew.-% | - | 1,0 Gew.-% | anderen Metallen, Metallsalzen und Nichtmetallen, welche allgemein als Verunreinigungen bezeichnet werden. |

Es ist bevorzugt, wenn die hierin offenbarte Legierung 5,0 - 25,5 Gew.-%, bevorzugt 5,2 - 22,0 Gew.-%, weiter bevorzugt 5,4 - 20,0 Gew.-%, weiter bevorzugt 5,5 - 19,0 Gew.-%, weiter bevorzugt 5,6 - 18,0 Gew.-%, weiter bevorzugt 5,7 - 17,0 Gew.-%, weiter bevorzugt 7,0 - 17,0 Gew.-%, weiter bevorzugt 7,5 - 16,5 Gew.-%, weiter bevorzugt 5,8 - 16,0 Gew.-%, weiter bevorzugt 8,0 - 16,0 Gew.-%, weiter bevorzugt 5,9 - 15,0 Gew.-%, weiter bevorzugt 8,3 - 15,8 Gew.-%, weiter bevorzugt 8,5 - 15,5 Gew.-%, weiter bevorzugt 8,7 - 15,0 Gew.-%, weiter bevorzugt 6,0 - 14,0 Gew.-%, weiter bevorzugt 8,8 - 14,8 Gew.-%, weiter bevorzugt 8,9 - 14,5 Gew.-%, weiter bevorzugt 9,0 - 14,0 Gew.-%, weiter bevorzugt 6,1 - 13,0 Gew.-%, weiter bevorzugt 9,1 - 13,5 Gew.-%, weiter bevorzugt 9,2 - 13,0 Gew.-%, weiter bevorzugt 6,2 - 12,5 Gew.-%, weiter bevorzugt 9,3 - 12,7 Gew.-%, weiter bevorzugt 9,4 - 12,4 Gew.-%, weiter bevorzugt 6,3 - 12,0 Gew.-%, weiter bevorzugt 9,5 - 12,2 Gew.-%, weiter bevorzugt 9,5 - 12,0 Gew.-%, weiter bevorzugt 6,4 - 11,5 Gew.-%, weiter bevorzugt 9,5 - 11,5 Gew.-%, weiter bevorzugt 6,5 - 11,0 Gew.-% und weiter bevorzugt 9,5 - 11,0 Gew.-% Dysprosium aufweist.

Bevorzugt liegt die Masse an Neodym im Bereich von 0,0 - 8,0 Gew.-%, weiter bevorzugt 0,1 - 5,0 Gew.-%, noch weiter bevorzugt 0,2 - 4,0 Gew.-%, noch weiter bevorzugt 0,3 - 3,0 Gew.-%, noch weiter bevorzugt 0,4 - 2,0 Gew.-% und insbesondere bevorzugt von 0,5 - 1,5 Gew.-%.

Zusammen mit Neodym (Nd) oder anstelle von Nd kann auch Europium (Eu) in der Legierung in Anteilen von 0,0 - 8,0 Gew.-%, weiter bevorzugt 0,1 - 5,0 Gew.-%, noch weiter bevorzugt 0,2 - 4,0 Gew.-%, noch weiter bevorzugt 0,3 - 3,0 Gew.-%, noch weiter bevorzugt 0,4 - 2,0 Gew.-% und insbesondere bevorzugt von 0,5 - 1,5 Gew.-% enthalten sein.

Ferner ist bevorzugt, wenn der gemeinsame Anteil an Nd und Eu in der Legierung von 0,01 - 8,0 Gew.-%, weiter bevorzugt 0,1 - 5,0 Gew.-%, noch weiter bevorzugt 0,2 - 4,0 Gew.-%, noch weiter bevorzugt 0,3 - 3,0 Gew.-%, noch weiter bevorzugt 0,4 - 2,0 Gew.-% und insbesondere bevorzugt von 0,5 - 1,5 Gew.-% beträgt.

Der Summe der Gewichtsanteile an Dysprosium und Neodym liegen bevorzugt im Bereich von 5,1 - 23,0 Gew.-%, weiter bevorzugt zwischen 6,6 - 15,5 Gew.-%, noch weiter bevorzugt, und insbesondere bevorzugt von 8,4 - 13,0 Gew.-%.

Ferner ist bevorzugt, wenn die hierin offenbarte Legierung zudem 0,2 - 4,0 Gew.-%, weiter bevorzugt 0,3 - 3,0 Gew.-%, noch weiter bevorzugt 0,4 - 2,0 Gew.-%, noch weiter bevorzugt 0,5 - 1,5 Gew.-% und insbesondere bevorzugt von 0,7 - 1,3 Gew.-% Zink (Zn) aufweist.

Zusätzlich zu den zuvor genannten Bestandteilen kann eine hierin beschriebene Magnesiumlegierung auch 0,0 Gew.-% - 1,0 Gew.-%, bevorzugt 0,1 Gew.-% - 0,6 Gew.-%, weiter bevorzugt 0,2 Gew.-% - 0,4 Gew.-% und besonders bevorzugt insgesamt nicht mehr als 0,3 Gew.-% andere Metalle, Metallsalze, Nichtmetalle, Kohlenstoff, Schwefel, Silizium, Stickstoff, Sauerstoff und/oder Wasserstoff enthalten. Diese weiteren Bestandteile sind Verunreinigungen, welche in den vorgenannten geringen Mengen unschädlich für die Produkteigenschaften oder die Eigenschaften der Legierung sind. Dies sind im Wesentlichen Fe und Si, die bei der Erzeugung des Primärmagnesiums durch den notwendigen Einsatz von FeSi beim Pidgeon-Prozess bzw. die allgemeine Verwendung von Stahl-Werkzeugen bei der Ver- und Bearbeitung von Magnesium und den Magnesiumlegierungen eingetragen werden können. Bevorzugt ist jedoch, dass die Metalle Cu, Ni, Fe jeweils unter 300 ppm, bevorzugt unter 200 ppm und weiter bevorzugt unter 150 ppm vorhanden sind. Die Schwermetalle, insbesondere Fe, Cu und Ni als edlere Bestandteile, bilden mit Magnesium ein galvanisches Element und erhöhen somit die Korrosion, insbesondere im Kontakt mit einem Korrosionsmittel wie dem Blut, in welchem Chlorid-Ionen vorliegen. Es entsteht im wässrigen Medium Wasserstoff, so dass eine Spannungsrisskorrosion entsteht, die bei Implantaten und insbesondere vaskulären Implantaten, wie Stents, vermieden werden soll. Cu, Ni, Fe verschlechtern somit das Korrosionsverhalten extrem, wenn mehr als die genannten Mengen vorhanden sind. Cu und Ni kommen in der Regel über Recyclingprozesse in die Mg-Legierungen und können vermieden werden, wenn man sauberes Primärmagnesium einsetzt.

Silizium (Si) sollte nicht in Mengen über 0,4 Gew.-%, bevorzugt über 0,3 Gew.-% und weiter bevorzugt über 0,2 Gew.-% vorhanden sein, weil Si die Legierungseigenschaften und Produkteigenschaften negativ beeinflusst, so verschlechtert die Zugabe von Silicium die Giessbarkeit. Es können sich stabile Silizide ($Mg_2Si$) bilden. Mit steigendem Anteil an $Mg_2Si$-Ausscheidungen versprödet das Material. $Mg_2Si$ bildet sich zudem nadelförmig aus und hat damit eine hohe Kerbwirkung und eine geringe Bruchdehnung zur Folge. Eine hohe Bruchdehnung ist jedoch für Stents notwendig und ein Krebsrisiko sollte zwingend vermieden werden.

Bevorzugt ist zudem, dass die Elemente Beryllium, Aluminium und Mangan jeweils unter 300 ppm, bevorzugt unter 200 ppm und weiter bevorzugt unter 150 ppm vorhanden sind. Beryllium, Berylliumoxid und Berylliumsalze sind für den Menschen giftig und werden als karzinogen eingestuft. Beryllium kann zu Haut-, Lungen-, Milz- und Leberschäden führen. Beryllium akkumuliert vor allem in Knochen, der Niere und in den Zellen des retikuloendothelialen Systems von Leber, Milz und Lymphknoten und führt nach jahrelanger Latenzzeit zur Bildung von Tumoren. Daher sollte Beryllium in einem degradierbaren vaskulären Implantat möglichst ganz vermieden werden. Es ist daher bevorzugt, dass die Legierung kein Beryllium enthält. Mangan als Spurenelement ist für den Menschen essentiell und ein wichtiger Bestandteil von Enzymen. Weiterhin wirkt Mangan jedoch auch neurotoxisch und schädigt das Zentralnervensystem. Durch eine chronisch exzessive Langzeit-Exposition kann es zu einer Demenzerkrankung mit Parkinsonähnlichen Symptomen wie motorischen Störungen kommen. Auch eine Rolle von Aluminium bei Alzheimer wird angenommen, Aluminium soll den Ausbruch der Alzheimer-Krankheit beschleunigen, wenn nicht gar verursachen. Zumindest wurde Aluminium in den Plaques im Gehirn von Alzheimer-Patienten nachgewiesen. Daher sollten Mangan und Aluminium als Bestandteile in einem degradierbaren vaskulären Implantat, welches sich über einen längeren Zeitraum zersetzt auch aus dem Aspekt der Vermarktung vollkommen vermieden werden.

Als weitere Metalle oder Nichtmetalle, welche einzeln oder zusammen in einer Maximalmenge von 0,3 Gew.-%, bevorzugt 0,2 Gew.-% und weiter bevorzugt 0,1 Gew.-% in der Legierung enthalten sein dürfen, zählen La, Ce, Pr und Sm. Hingegen sollten folgende Elemente vermieden oder zusammen in einer Maximalmenge von 0,1 Gew.-%, bevorzugt 0,05 Gew.-%, weiter bevorzugt 0,01 Gew.-%, noch weiter bevorzugt 0,005 Gew.-% und noch weiter bevorzugt 0,001 Gew.-% in der Legierung enthalten sein: Tb, Ho, Er, Tm, Yb und Lu. Es ist also bevorzugt, dass die hierin beschriebenen Magnesiumlegierungen und die daraus hergestellten Stents insgesamt nicht mehr als 0,1 Gew.-% bevorzugt 0,05 Gew.-% und weiter bevorzugt 0,01 Gew.-% der Elemente Terbium, Holmium, Erbium, Thulium, Ytterbium und Lutetium enthalten, wobei weiter bevorzugt diese Elemente ganz vermieden werden sollten, d.h. als Verunreinigung im ppm-Bereich jeweils unterhalb von 150 ppm und insbesondere bevorzugt unterhalb von 100 ppm liegen sollten.

In der Maximalmenge von 1,0 Gew.-% Verunreinigungen sind die weiteren Metalle oder Nichtmetalle wie z.B. Silizium, Kohlenstoff, Sauerstoff, Stickstoff, Wasserstoff oder Schwefel enthalten, auch wenn diese zusätzlich explizit aufgeführt sind.

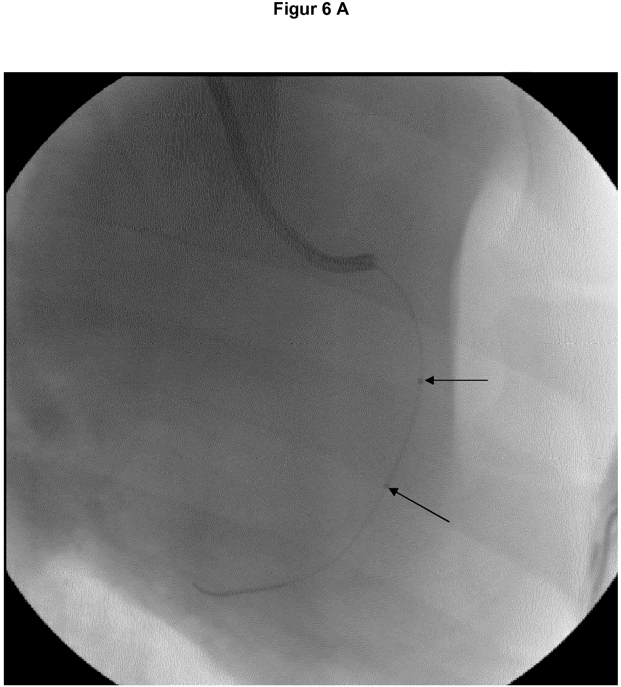

Es wurde überraschend festgestellt, dass trotz der relativ großen Menge an Dysprosium, die hierin beschriebenen Legierungen, bzw. die daraus hergestellten Stents nicht röntgendicht sind. Die im Rahmen der Tierversuchsstudie durchgeführte Angiographie zeigte, dass die Stents nicht opak waren, das heißt, dass sie in den Röntgenaufnahmen während der Koronarangiographie nicht zu sehen waren (s. Figur 6A - 6D). Dies erlaubt es, dass man das Gefäßlumen mittels Kontrastmittelapplikation deutlich sehen kann. Dadurch kann man den Verlauf der Heilung und die Kontrolle bezüglich möglicher In-Stent-Restenosen bei den Patienten nicht-invasiv verfolgen, d.h. mit bildgebenden Verfahren, wie MRT oder CT. Es ist daher bevorzugt, dass die hierin beschriebenen Legierungen nicht röntgendicht oder nicht radio-opak sind. Sofern die Stents dennoch im Röntgenbild sichtbar sein sollen, so dass die korrekte Platzierung kontrolliert werden kann, könnte man an bestimmten Stellen des Stents oder des Katheterballons, welcher zur Implantation des Stents verwendet wird, radio-opake oder röntgendichte Marker anbringen, was in der Praxis auch regelmäßig geschieht.

Eine bevorzugte Zusammensetzung einer hierin beschriebenen Magnesiumlegierung umfasst oder besteht aus:

| | | | |
|---|---|---|---|
| 77,0 Gew.-% | - | 94,6 Gew.-% | Magnesium |
| 5,0 Gew.-% | - | 15,0 Gew.-% | Dysprosium |
| 0,2 Gew.-% | - | 4,0 Gew.-% | Neodym |
| 0,2 Gew.-% | - | 4,0 Gew.-% | Zink |

Eine bevorzugte Zusammensetzung einer hierin beschriebenen Magnesiumlegierung umfasst oder besteht aus:

| | |
|---|---|
| 88 Gew.-% | Magnesium |
| 10 Gew.-% | Dysprosium |
| 1 Gew.-% | Neodym |
| 1 Gew.-% | Zink |

Eine bevorzugte Zusammensetzung einer hierin beschriebenen Magnesiumlegierung umfasst oder besteht aus:

| | | | |
|---|---|---|---|
| 79,0 Gew.-% | - | 94,75 Gew.-% | Magnesium |
| 5,0 Gew.-% | - | 15,0 Gew.-% | Dysprosium |
| 0,2 Gew.-% | - | 4,0 Gew.-% | Neodym |
| 0,05 Gew.-% | - | 2,0 Gew.-% | Zirkonium |

Eine weitere, bevorzugte Zusammensetzung einer hierin beschriebenen Magnesiumlegierung umfasst oder besteht aus:

| | |
|---|---|
| 88 Gew.-% | Magnesium |
| 10 Gew.-% | Dysprosium |
| 1 Gew.-% | Neodym |
| 1 Gew.-% | Zirkonium |

Eine bevorzugte Zusammensetzung einer hierin beschriebenen Magnesiumlegierung umfasst oder besteht aus:

| | | | |
|---|---|---|---|
| 75,0 Gew.-% | - | 94,55 Gew.-% | Magnesium |
| 5,0 Gew.-% | - | 15,0 Gew.-% | Dysprosium |
| 0,2 Gew.-% | - | 4,0 Gew.-% | Neodym |
| 0,2 Gew.-% | - | 4,0 Gew.-% | Zink |
| 0,05 Gew.-% | - | 2,0 Gew.-% | Zirkonium |

Eine besonders bevorzugte Zusammensetzung einer hierin beschriebenen Magnesiumlegierung umfasst oder besteht aus:

| | |
|---|---|
| 87 Gew.-% | Magnesium |
| 10 Gew.-% | Dysprosium |
| 1 Gew.-% | Neodym |
| 1 Gew.-% | Zink |
| 1 Gew.-% | Zirkonium |

Eine bevorzugte Zusammensetzung einer hierin beschriebenen Magnesiumlegierung umfasst oder besteht aus:

| | | | |
|---|---|---|---|
| 76,0 Gew.-% | - | 94,5 Gew.-% | Magnesium |
| 5,0 Gew.-% | - | 15,0 Gew.-% | Dysprosium |
| 0,2 Gew.-% | - | 4,0 Gew.-% | Neodym |
| 0,2 Gew.-% | - | 4,0 Gew.-% | Zink |
| 0,1 Gew.-% | - | 1,0 Gew.-% | Verunreinigungen wie z.B. andere Metalle, Metallsalze und Nichtmetalle. |

Eine bevorzugte Zusammensetzung einer hierin beschriebenen Magnesiumlegierung umfasst oder besteht aus:

| | | | |
|---|---|---|---|
| 80,0 Gew.-% | - | 94,7 Gew.-% | Magnesium |
| 5,0 Gew.-% | - | 15,0 Gew.-% | Dysprosium |
| 0,1 Gew.-% | - | 2,0 Gew.-% | Neodym |
| 0,1 Gew.-% | - | 2,0 Gew.-% | Zink |
| 0,1 Gew.-% | - | 1,0 Gew.-% | Verunreinigungen wie z.B. andere Metalle, Metallsalze und Nichtmetalle. |

Eine weitere bevorzugte Zusammensetzung einer hierin beschriebenen Magnesiumlegierung umfasst oder besteht aus:

| | | | |
|---|---|---|---|
| 81,7 Gew.-% | - | 94,7 Gew.-% | Magnesium |
| 5,0 Gew.-% | - | 15,0 Gew.-% | Dysprosium |
| 0,1 Gew.-% | - | 2.0 Gew.-% | Neodym |
| 0,1 Gew.-% | - | 0,3 Gew.-% | Zirkonium |
| 0,1 Gew.-% | - | 1,0 Gew.-% | Verunreinigungen wie z.B. andere Metalle, Metallsalze und Nichtmetalle. |

Eine weitere bevorzugte Zusammensetzung einer hierin beschriebenen Magnesiumlegierung umfasst oder besteht aus:

| 79,7 Gew.-% | - | 94,6 Gew.-% | Magnesium |
|---|---|---|---|
| 5,0 Gew.-% | - | 15,0 Gew.-% | Dysprosium |
| 0,1 Gew.-% | - | 2,0 Gew.-% | Neodym |
| 0,1 Gew.-% | - | 2,0 Gew.-% | Zink |
| 0,1 Gew.-% | - | 0,3 Gew.-% | Zirkonium |
| 0,1 Gew.-% | - | 1,0 Gew.-% | Verunreinigungen wie z.B. andere Metalle, Metallsalze und Nichtmetalle. |

Eine weitere bevorzugte Zusammensetzung einer hierin beschriebenen Magnesiumlegierung umfasst oder besteht aus:

| 79,7 Gew.-% | - | 94,6 Gew.-% | Magnesium |
|---|---|---|---|
| 5,0 Gew.-% | - | 15,0 Gew.-% | Dysprosium |
| 0,1 Gew.-% | - | 2,0 Gew.-% | Europium |
| 0,1 Gew.-% | - | 2,0 Gew.-% | Zink |
| 0,1 Gew.-% | - | 0,3 Gew.-% | Zirkonium |
| 0,1 Gew.-% | - | 1,0 Gew.-% | Verunreinigungen wie z.B. andere Metalle, Metallsalze und Nichtmetalle. |

Eine weitere bevorzugte Zusammensetzung einer hierin beschriebenen Magnesiumlegierung umfasst oder besteht aus:

| 77,7 Gew.-% | - | 94,5 Gew.-% | Magnesium |
|---|---|---|---|
| 5,0 Gew.-% | - | 15,0 Gew.-% | Dysprosium |
| 0,1 Gew.-% | - | 2,0 Gew.-% | Europium |
| 0,1 Gew.-% | - | 2,0 Gew.-% | Neodym |
| 0,1 Gew.-% | - | 2,0 Gew.-% | Zink |
| 0,1 Gew.-% | - | 0,3 Gew.-% | Zirkonium |
| 0,1 Gew.-% | - | 1,0 Gew.-% | Verunreinigungen wie z.B. andere Metalle, Metallsalze und Nichtmetalle. |

Eine weitere bevorzugte Zusammensetzung einer hierin beschriebenen Magnesiumlegierung umfasst oder besteht aus:

| 79,0 Gew.-% | - | 94,75 Gew.-% | Magnesium |
|---|---|---|---|
| 5,0 Gew.-% | - | 15,0 Gew.-% | Dysprosium |
| 0,2 Gew.-% | - | 4,0 Gew.-% | Europium |
| 0,05 Gew.-% | - | 2,0 Gew.-% | Zirkonium |

Eine weitere bevorzugte Zusammensetzung einer hierin beschriebenen Magnesiumlegierung umfasst oder besteht aus:

| 88 Gew.-% | Magnesium |
|---|---|
| 10 Gew.-% | Dysprosium |
| 1 Gew.-% | Europium |
| 1 Gew.-% | Zirkonium |

Eine weitere bevorzugte Zusammensetzung einer hierin beschriebenen Magnesiumlegierung umfasst oder besteht aus:

| 77,0 Gew.-% | - | 94,75 Gew.-% | Magnesium |
|---|---|---|---|
| 5,0 Gew.-% | - | 15,0 Gew.-% | Dysprosium |
| 0,2 Gew.-% | - | 4,0 Gew.-% | Europium |
| 0,05 Gew.-% | - | 4,0 Gew.-% | Zink |

Eine weitere bevorzugte Zusammensetzung einer hierin beschriebenen Magnesiumlegierung umfasst oder besteht aus:

| | |
|---|---|
| 88 Gew.-% | Magnesium |
| 10 Gew.-% | Dysprosium |
| 1 Gew.-% | Europium |
| 1 Gew.-% | Zink |

Eine weitere bevorzugte Zusammensetzung einer hierin beschriebenen Magnesiumlegierung umfasst oder besteht aus:

| | | | |
|---|---|---|---|
| 75,0 Gew.-% | - | 94,7 Gew.-% | Magnesium |
| 5,0 Gew.-% | - | 15,0 Gew.-% | Dysprosium |
| 0,2 Gew.-% | - | 4,0 Gew.-% | Europium |
| 0,05 Gew.-% | - | 4,0 Gew.-% | Zink |
| 0,05 Gew.-% | - | 2,0 Gew.-% | Zirkonium |

Eine weitere bevorzugte Zusammensetzung einer hierin beschriebenen Magnesiumlegierung umfasst oder besteht aus:

| | |
|---|---|
| 87 Gew.-% | Magnesium |
| 10 Gew.-% | Dysprosium |
| 1 Gew.-% | Europium |
| 1 Gew.-% | Zink |
| 1 Gew.-% | Zirkonium |

Eine besonders bevorzugte Zusammensetzung einer hierin beschriebenen Magnesiumlegierung umfasst oder besteht aus:

| | |
|---|---|
| 87,8 Gew.-% | Magnesium |
| 10,0 Gew.-% | Dysprosium |
| 1,0 Gew.-% | Neodym |
| 1,0 Gew.-% | Zink |
| 0,2 Gew.-% | Zirkonium |

Eine weitere besonders bevorzugte Zusammensetzung einer hierin beschriebenen Magnesiumlegierung umfasst oder besteht aus:

| | |
|---|---|
| 87,8 Gew.-% | Magnesium |
| 10,0 Gew.-% | Dysprosium |
| 1,0 Gew.-% | Europium |
| 1,0 Gew.-% | Zink |
| 0,2 Gew.-% | Zirkonium |

Eine weitere besonders bevorzugte Zusammensetzung einer hierin beschriebenen Magnesiumlegierung umfasst oder besteht aus:

| | |
|---|---|
| 86,8 Gew.-% | Magnesium |
| 10,0 Gew.-% | Dysprosium |
| 1,0 Gew.-% | Neodym |
| 1,0 Gew.-% | Europium |
| 1,0 Gew.-% | Zink |
| 0,2 Gew.-% | Zirkonium |

Alle in dieser Offenbarung angegebenen Gew.-% beziehen sich auf das Gesamtgewicht der entsprechenden Legierung. Somit gilt bei allen hierin aufgeführten Zusammensetzungen, dass die Summe aller Bestandteile insgesamt 100,00 Gew.-% ergeben muss. Das heißt, dass nach Addition aller aufgeführter Komponenten der Magnesiumlegierung die Differenz zu 100 Gew.-% aus Magnesium als Hauptbestandteil besteht. Darüber hinaus können diese Zusammensetzungen noch einen sehr geringen Anteil an teilweise nicht vermeidbaren, herstellungsbedingten Verunreinigungen enthalten. Es ist bevorzugt, dass diese Verunreinigungen jeweils ≤ 0,2 Gew.-%, bevorzugt ≤ 0,02 Gew.-% und insbesondere bevorzugt ≤ 250 ppm und in der Summe aller Verunreinigungen ≤ 0,4 Gew.-%, bevorzugt ≤ 0,05 Gew.-% und insbesondere bevorzugt ≤ 150 ppm sind. Es ist besonders bevorzugt, wenn die unvermeidbaren Verunreinigungen weniger als 0,1 Gew.-% bevorzugt 0,05 Gew.-% und weiter bevorzugt 0,01 Gew.-%, weiter bevorzugt unterhalb von 150 ppm und insbesondere bevorzugt unterhalb von 100 ppm betragen. Handelt es sich bei den Verunreinigungen um Metalle, Metallsalze oder Metallcarbide, Metallnitride, Metalloxide, Metallsilikate oder Metallsilicide so ist bevorzugt, dass derartige Verunreinigungen jeweils in Mengen von unterhalb 300 ppm, bevorzugt 200 ppm und weiter bevorzugt unterhalb von 150 ppm vorliegen. Der Begriff "Verunreinigungen" wie hierin verwendet bezeichnet alle Legierungsbestandteile außer Magnesium, Dysprosium, Neodym, Europium, Zink und Zirkonium ungeachtet, ob diese explizit aufgeführt sind oder nicht.

Ferner umfasst die vorliegende Erfindung bevorzugt Magnesiumlegierungen, die neben Magnesium, Dysprosium, Neodym, Europium, Zink, Zirkonium und nicht vermeidbaren, herstellungsbedingten Verunreinigungen keine weiteren Bestandteile umfassen. Das heißt, es ist bevorzugt, wenn die Bestandteile der Legierung, neben der Basis Magnesium, ausgewählt werden aus der folgenden Gruppe bestehend aus oder umfassend: Dysprosium, Neodym, Europium, Zink, Zirkonium und nicht vermeidbaren, herstellungsbedingten Verunreinigungen. Es ist insbesondere bevorzugt, dass die Legierungen kein Yttrium enthalten. Kein Yttrium bedeutet maximal 150 ppm Yttrium. Yttrium ist für Zellen *in vitro* weniger tolerabel als andere seltene Erden wie z.B. Dysprosium. Zudem ist die Wirksamkeit von Yttrium zur Festigkeitssteigerung über Mischkristall- bzw. Ausscheidungshärtung deutlich geringer als die anderer seltener Erden, wie z.B. Dysprosium, da Yttrium eine deutlich geringere Löslichkeit in Magnesium hat als Dysprosium (Löslichkeit in Magnesium: Y:12,47 Gew.-%, Dy:25,34 Gew.-%). Freie Gadoliniumionen sind hochgiftig. Wegen der hohen Giftigkeit von freien Gadoliniumionen werden diese in Kontrastmitteln nur mit Komplexierungsmitteln mit hoher Komplexierungskonstante (z.B. der Chelator DTPA) eingesetzt. Es wird heute jedoch davon ausgegangen, dass bei Patienten mit stark eingeschränkter Nierenfunktion, Dialysepatienten und Lebertransplantierten die Freisetzung von hochtoxischen freien Gadoliniumionen aus Kontrastmitteln die Entstehung von nephrogener, systemischer Fibrose (NSF) verursacht. Lithium wird bei psychischen Erkrankungen, vor allem bei affektiven Störungen, in Form bestimmter Salze als Medikament eingesetzt. Allerdings besitzt Lithium eine geringe therapeutische Breite und schon ab einem Serumspiegel von 1,5 mmol/l kann es zu Nebenwirkungen kommen. Unter Langzeit-Behandlung mit Lithium können Wasser- und Natriumverluste (Diabetes insipidus), Übersäuerung des Blutes (Azidose) und eine Lithium-Nephropathie mit Einschränkung der Nierenfunktion auftreten. Eines der Probleme ist hier, dass der $Li^+$-Plasmaspiegel und damit die Lithiumwirkung von allen Substanzen und äußeren Umständen mit Wirkung auf die $Na^+$-Ausscheidung beeinflusst wird. Daher liegt in der Freisetzung von Lithiumionen eine potentielle Gefahr von unerwünschten Nebenwirkungen.

Des Weiteren ist es daher bevorzugt, wenn die Zusammensetzung der Magnesiumlegierung kein Lithium und/oder kein Gadolinium enthält. Diese Metalle sind vorzugsweise nicht oder nur in geringen Mengen von insgesamt 0,01 Gew.-% - 0,1 Gew.-%, bevorzugt in Mengen von insgesamt 0,001 Gew.-% - 0,01 Gew.-% enthalten. Weiter bevorzugt sind Li und Gd nur als Verunreinigungen im ppm-Bereich unterhalb von 150 ppm insbesondere bevorzugt unterhalb von 100 ppm enthalten.

Falls weitere Bestandteile neben Magnesium, Dysprosium, Neodym, Europium, Zink und Zirkonium in der Legierung enthalten sind, so handelt es sich bei diesen weiteren Bestandteilen um Verunreinigungen wie z.B. andere Metalle, Metallsalze, Nichtmetalle, Kohlenstoff, Schwefel, Stickstoff, Sauerstoff, Silizium und/oder Wasserstoff, welche zusammen in geringen Mengen von < 0,6 Gew.-%, bevorzugt < 0,5 Gew.-%, weiter bevorzugt < 0,4 Gew.-%, weiter bevorzugt < 0,3 Gew.-%, weiter bevorzugt < 0,2 Gew.-% und insbesondere bevorzugt < 0,1 Gew.-% vorhanden sind.

Als "andere Metalle", welche in der Zusammensetzung der hierin beschriebenen Magnesiumlegierung vorhanden sein können, sind die folgenden zu nennen: Natrium, Kalium, Calcium, Scandium, Titan, Vanadium, Chrom, Eisen, Cobalt, Nickel, Kupfer, Gallium, Niob, Molybdän, Technetium, Ruthenium, Rhodium, Palladium, Silber, Indium, Zinn, Promethium, Tantal, Wolfram, Rhenium, Platin, Gold und Blei. Außer Lanthan, Cer, Praseodym und Samarium, welche jeweils bis maximal 0,3 Gew.-%, bevorzugt 0,2 Gew.-% und weiter bevorzugt maximal 0,1 Gew.-% in der Legierung enthalten sein dürfen und die Elemente Strontium, Natrium, Kalium, Calcium, Scandium, Titan, Vanadium, Chrom, Cobalt, Gallium, Niob, Molybdän, Technetium, Ruthenium, Rhodium, Palladium, Silber, Indium, Zinn, Promethium, Tantal, Wolfram, Rhenium, Platin, Gold und Blei, welche vorzugsweise nur als Verunreinigungen im ppm-Bereich vorliegen und jeweils eine Menge von 500 ppm, bevorzugt 400 ppm, weiter bevorzugt 300 ppm, noch weiter bevorzugt 200 ppm und insbesondere bevorzugt 150 ppm nicht überschreiten sollten.

Ferner können Metallsalze in sehr geringen Mengen in der Legierung enthalten sein. Salze von Fe, Cu, Ni oder Co dürfen nur in Mengen bis zum 100 ppm vorzugsweise nur bis zu 50 ppm enthalten sein. Die Elemente Terbium, Holmium, Erbium, Thulium, Ytterbium, Lutetium, Beryllium, Aluminium, Mangan, Kupfer, Nickel, Eisen, Lithium und Gadolinium sollten vorzugsweise jeweils in Mengen geringen als 300 ppm, bevorzugt 200 ppm, weiter bevorzugt 150 ppm und insbesondere bevorzugt jeweils 100 ppm in der Legierung enthalten sein und zusammen eine Menge von 3000 ppm, bevorzugt 2000 ppm, weiter bevorzugt 1500 ppm und insbesondere bevorzugt jeweils 1000 ppm nicht überschreiten.

Metallsalze enthalten vorzugsweise mindestens eines der folgenden Metallionen: $Na^+$, $Mg^{2+}$, $K^+$, $Ca^{2+}$, $Sc^{3+}$, $Ti^{2+}$, $Ti^{4+}$, $V^{2+}$, $V^{3+}$, $V^{4+}$, $V^{5+}$, $Cr^{2+}$, $Cr^{3+}$, $Cr^{4+}$, $Cr^{6+}$, $Fe^{2+}$, $Fe^{3+}$, $Co^{2+}$, $Co^{3+}$, $Ni^{2+}$, $Cu^+$, $Cu^{2+}$, $Zn^{2+}$, $Zr^{2+}$, $Zr^{4+}$, $Nb^{2+}$, $Nb^{4+}$, $Nb^{5+}$, $Mo^{4+}$, $Mo^{6+}$, $Tc^{2+}$, $Tc^{3+}$, $Tc^{4+}$, $Tc^{5+}$, $Tc^{6+}$, $Tc^{7+}$, $Ru^{3+}$, $Ru^{4+}$, $Ru^{5+}$, $Ru^{6+}$, $Ru^{7+}$, $Ru^{8+}$, $Rh^{3+}$, $Rh^{4+}$, $Pd^{2+}$, $Pd^{3+}$, $Ag^+$, $In^+$, $In^{3+}$, $Ta^{4+}$, $Ta^{5+}$, $W^{4+}$, $W^{6+}$, $Pt^{2+}$, $Pt^{3+}$, $Pt^{4+}$, $Pt^{5+}$, $Pt^{6+}$, $Au^+$, $Au^{3+}$, $Au^{5+}$, $Sn^{2+}$, $Sn^{4+}$, $Pb^{2+}$, $Pb^{4+}$, $La^{3+}$, $Ce^{3+}$, $Ce^{4+}$, $Gd^{3+}$, $Nd^{3+}$, $Pr^{3+}$, $Pr^{3+}$, $Pm^{3+}$, $Sm^{3+}$, $Eu^{2+}$, $Dy^{3+}$.

Als Anionen dienen Halogene wie $F^-$, $Cl^-$, $Br^-$, Oxide und Hydroxide wie $OH^-$, $O^{2-}$, Sulfate, Carbonate, Oxalate, Phosphate wie $HSO_4^-$, $SO_4^{2-}$, $HCO_3^-$, $CO_3^{2-}$, $HC_2O_4^-$, $C_2O_4^{2-}$, $H_2PO_4^-$, $HPO_4^{2-}$, $PO_4^{3-}$.

Die erfindungsgemäßen Magnesiumlegierungen sind insbesondere zur Herstellung von resorbierbaren oder degradierbaren Endoprothesen und insbesondere vaskulären Implantaten bzw. Stents geeignet.

Des Weiteren umfasst die vorliegende Erfindung daher einen resorbierbaren Stent oder ein resorbierbares, vaskuläres Implantat bestehend aus einer der hier offenbarten Magnesiumlegierungen. Es handelt sich bei dem resorbierbaren Stent bevorzugt um einen Stent für Blutgefäße, Harnwege, Atemwege, Gallenwege oder den Verdauungtrakt. Unter diesen Stents sind wiederum die vaskulären Implantate oder Stents für Blutgefäße oder allgemeiner für das Herz-Kreislaufsystem bevorzugt.

Der Begriff "resorbierbar" wie hierin verwendet bedeutet, dass das Implantat sich über eine gewisse Zeit in einem menschlichen oder tierischen Organismus langsam auflöst und irgendwann nur noch dessen Abbauprodukte im Körper in gelöster Form vorliegen. Zu diesem Zeitpunkt sind feste Bestandteile oder Fragmente des Implantats nicht mehr vorhanden. Die Abbauprodukte sollten physiologisch weitgehend unbedenklich sein und zu Ionen oder Molekülen führen, welche im Organismus sowieso vorhanden oder vom Organismus zu unbedenklichen Stoffen abgebaut oder ausgeschieden werden können.

Die Stents bzw. vaskulären Implantate werden vorzugsweise per Laser aus einem Rohr geschnitten, welches aus einer hierin beschriebenen Magnesiumlegierung besteht. Die erfindungsgemäßen Stents werden innerhalb eines Zeitraumes von 8 bis 50 Wochen, vorzugsweise 10 bis 30 Wochen unter physiologischen Bedingungen resorbiert.

Die Begriffe "resorbierbar" oder "degradierbar" oder "bioabbaubar" oder "biologisch abbaubar" bezeichnen also den Sachverhalt, dass der menschliche oder tierische Körper in der Lage ist, den Stent oder die vaskulären Implantate innerhalb eines gewissen Zeitraums aufzulösen, so dass Atome, Ionen oder Moleküle vorliegen, welche im Blut oder anderen Körperflüssigkeiten gelöst vorliegen können.

Unter Stents werden hierbei gitterförmige oder netzförmige Endoprothesen verstanden, die in ein Hohlorgan oder einen Körperhohlraum eingesetzt werden, um diesen offen zu halten. Ein Stent bildet kein massives Rohr, sondern ein Gittergeflecht. Betrachtet man beispielsweise einen vaskulären Stent, so wird dieser aus einem massiven Rohr z.B. mittels Laser geschnitten, so dass sich einzelne, möglichst dünne Streben ergeben, welche untereinander verbunden sind. Die Anordnung und Ausformung der Streben und Knotenpunkte wird als Stentdesign bezeichnet.

Beim Schneiden eines Stents werden Flächen zwischen den einzelnen Streben herausgeschnitten. Ein Stent oder ein vaskuläres Implantat weist daher eine Vielzahl von massiven Gerüstkomponenten (z.B. Streben in Form von Ringen, Spiralen, Wellen und Drähten) auf, welche insgesamt die Endoprothese oder den Stent bilden, sowie eine Vielzahl von Zwischenräumen zwischen diesen massiven Komponenten. Bei der gängigen Ausführungsform von Endoprothesen oder Stents laufen die Streben in Knotenpunkten zusammen. Es gibt jedoch auch Ausführungsformen von Endoprothesen, bei denen keine oder fast keine Knotenpunkte vorhanden sind und die Streben z.B. die Form von Ringen oder Spiralen haben. Bevorzugt handelt es sich um selbstexpandierbare oder ballonexpandierbare Stents, welche mittels Katheter bis zur erkrankten oder zu behandelnden Stelle geschoben werden, wo die Stents auf ihren definierten Normdurchmesser aufgeweitet werden.

Die vaskulären Implantate oder Stents werden aus Rohren mittels Laser geschnitten, die aus einer hierin beschriebenen Legierung bestehen. Die Rohre werden durch Umformung von Drähten aus den hierin beschriebenen Legierungen erhalten. Die hierin beschriebenen Legierungen werden bevorzugt im sogenannten "Tütengußverfahren" hergestellt. Bei diesem Verfahren werden die Komponenten der Magnesiumlegierungen in einem geschlichteten Stahltiegel unter Schutzgasatmosphäre bei einer Schmelzbadtemperatur von 660 bis 740°C erschmolzen. Die Schmelze wird so lange gerührt, bis eine vollständige Durchmischung stattgefunden hat und danach in eine dünnwandige Kokille überführt, die auf eine Temperatur von 600°C vorgeheizt wurde und für eine Stunde bei einer Temperatur von ca. 700°C gehalten. Danach wird die Kokille im Wasserbad einer Temperatur von 15 - 20°C abgekühlt. Die erhaltenen Bolzen werden dann vor dem Strangpressen auf eine Temperatur von 250-500°C aufgeheizt und für 3 - 6 Stunden bei dieser Temperatur gehalten. Es folgt das Strangpressen und die Abkühlung des gepressten Stranges auf Raumtemperatur.

Hierin beschrieben werden nach dem Tütengußverfahren erhältliche Legierungen. Besonders bevorzugt ist eine nach dem Tütengußverfahren erhaltene Magnesiumlegierung die bezogen auf das Gesamtgewicht der Legierung folgende Bestandteile enthält:

| | | | |
|---|---|---|---|
| 80,4 Gew.-%- 94,6 Gew.-% | | | Magnesium |
| 5,0 Gew.-% | - | 15,0 Gew.-% | Dysprosium |
| 0,1 Gew.-% | - | 2,0 Gew.-% | Neodym und/oder Europium |
| 0,1 Gew.-% | - | 2,0 Gew.-% | Zink |
| 0,1 Gew.-% | - | 0,3 Gew.-% | Zirkonium |

und Verunreinigungen wie z.B. andere Metallen, Metallsalzen und Nichtmetallen in den hierin offenbarten Mengen, wobei die Legierung kein Yttrium und kein Gadolinium enthält.

Hierin offenbart werden auch alle weiteren nach dem Tütengußverfahren erhaltenen Magnesiumlegierungen mit einer der hierin offenbarten Zusammensetzungen. Daher gelten die offenbarten, bevorzugten Bereiche der einzelnen Legierungselemente auch im Zusammenhang mit nach dem Tütengußverfahhren erhaltenen Magnesiumlegierungen.

Ferner beschrieben werden Drähte, welche vorzugsweise durch Strangpressen aus den nach dem Tütengußverfahren erhältlichen, Legierungen hergestellt werden. Die hierin offenbarten, bevorzugten Mengenbereiche der einzelnen Legierungselemente gelten auch im Zusammenhang, mit Drähten aus nach dem Tütengußverfahren erhaltenen Magnesiumlegierungen. Beschrieben werden also insbesondere Drähte erhältlich aus einer nach dem Tütengußverfahren erhaltenen Magnesiumlegierung die bezogen auf das Gesamtgewicht der Legierung folgende Bestandteile enthält:

| | | | |
|---|---|---|---|
| 80,4 Gew.-%- 94,6 Gew.-% | | | Magnesium |
| 5,0 Gew.-% | - | 15,0 Gew.-% | Dysprosium |
| 0,1 Gew.-% | - | 2,0 Gew.-% | Neodym und/oder Europium |
| 0,1 Gew.-% | - | 2,0 Gew.-% | Zink |
| 0,1 Gew.-% | - | 0,3 Gew.-% | Zirkonium |

und Verunreinigungen wie z.B. andere Metallen, Metallsalzen und Nichtmetallen in den hierin offenbarten Mengen, wobei die Legierung kein Yttrium und kein Gadolinium enthält und wobei die Drähte durch Strangpressen erhalten wurden.

Zudem beschrieben werden Rohre, welche aus den vorzugsweise durch Strangpressen erhältlichen Drähten, welche aus den nach dem Tütengußverfahren erhältlichen, Legierungen bestehen, hergestellt werden.

Die hierin offenbarten, bevorzugten Mengenbereiche der einzelnen Legierungselemente gelten auch im Zusammenhang, mit Rohren, hergestellt aus Drähten bestehend aus nach dem Tütengußverfahren erhaltenen Magnesiumlegierungen.

Beschrieben werden also insbesondere Rohre erhältlich aus einer nach dem Tütengußverfahren erhaltenen Magnesiumlegierung die bezogen auf das Gesamtgewicht der Legierung folgende Bestandteile enthält:

| | | | |
|---|---|---|---|
| 80,4 Gew.-%- 94,6 Gew.-% | | | Magnesium |
| 5,0 Gew.-% | - | 15,0 Gew.-% | Dysprosium |
| 0,1 Gew.-% | - | 2,0 Gew.-% | Neodym und/oder Europium |
| 0,1 Gew.-% | - | 2,0 Gew.-% | Zink |
| 0,1 Gew.-% | - | 0,3 Gew.-% | Zirkonium |

und Verunreinigungen wie z.B. andere Metallen, Metallsalzen und Nichtmetallen in den hierin offenbarten Mengen, wobei die Legierung kein Yttrium und kein Gadolinium enthält und wobei die Rohre erhältlich sind aus Drähten, die durch Strangpressen erhalten wurden.

Die vorliegende Erfindung betrifft zudem Stents, welche aus den Rohren geschnitten werden, wobei die Rohre aus den vorzugsweise durch Strangpressen erhältlichen Drähten hergestellt werden, wobei die Drähte aus den nach dem Tütengußverfahren erhältlichen, hierin offenbarten Legierungen bestehen. Die hierin offenbarten, bevorzugten Mengenbereiche der einzelnen Legierungselemente gelten auch im Zusammenhang, mit Stents, hergestellt aus Rohren, welche wiederum aus Drähten bestehend aus nach dem Tütengußverfahren erhaltenen Magnesiumlegierungen geformt wurden.

Beschrieben wird zudem ein Verfahren zur Herstellung von resorbierbaren Stents, umfassend die folgende Schritte:

a) Bereitstellung einer nach dem Tütengußverfahren erhältlichen, hierin offenbarten Legierung,
b) Herstellung eines Drahtes aus der Legierung erhalten gemäß Schritt a) durch Strangpressen,

c) Herstellung eines Rohres aus dem Draht erhalten gemäß Schritt b), und
d) Schneiden von Stents aus dem Rohr erhalten gemäß Schritt c).

Beschrieben werden auch resorbierbare Stents erhalten nach obigem Verfahren. In Schritt d) wird bevorzugt ein Laser verwendet, um die Stents aus dem Rohr erhalten gemäß Schritt c) zu schneiden. Um eine Magnesiumlegierung nach dem Tütengußverfahren zu erhalten können vor Schritt a) noch weitere Schritte durchgeführt werden. Bei diesen Schritten werden in einem geschlichteten Stahltiegel bei einer Schmelzbadtemperatur von 660-740°C durch schrittweise zugeben der Legierungselemente in Form von reinen Elementen oder als Masterlegierungen in einem geschlichteten Stahltiegel erschmolzen. Als Tiegelwerkstoff kann praktisch jeder nickelfreie Stahl verwendet werden. Graphit wäre eine weitere Möglichkeit. Alle Schmelzoperationen finden unter Schutzgas statt. Nach der Zugabe der Legierungselemente wird die Schmelze mechanisch gerührt. In einem nächsten Schritt wird diese Schmelze in eine dünnwandige Kokille überführt, die auf eine Temperatur von 600°C vorgeheizt wurde. In einem letzten Schritt wird die Kokille in ein Wasserbad eingetaucht, das eine Temperatur von 15-20°C aufweist.

In einer tierexperimentellen Studie (s. Beispiel 7) zur Wirksamkeit und Unbedenklichkeit von Stents aus hierin beschriebenen Magnesiumlegierungen konnte gezeigt werden, dass die erfindungsgemäßen Stents bzw. vaskuläre Implantate aus den hier offenbarten Magnesiumlegierungen sich ohne Probleme auf einen Ballon crimpen lassen. Die Implantation der Stents erfolgte ohne das Auftreten von bekannten Komplikationen, wie Stentmalapposition, Thrombosen oder Dissektion. Bereits nach 4 Wochen war eine vollständige Reendothelialisierung der gestenteten Gefäßabschnitte zu beobachten. Dies spricht dafür, dass keine übermäßigen Endzündungsreaktionen stattfanden und die Magnesiumlegierungen keine Unverträglichkeitsreaktionen im Gewebe des Gefäßes hervorgerufen haben. Die Restenoserate lag im Bereich der Werte von herkömmlichen Metallstents (BMS) im Stand der Technik, bzw. im Bereich der "schlechteren" Wirkstoff-freisetzenden Stents (DES) (vgl. Abbildungen zum Vortrag R.A. Costa; gehalten i.R. des Euro-PCR, Paris, im Mai 2011).

Figurenbeschreibung

Figur 1: zeigt eine Aufnahme zur Bestimmung der Korngröße einer Magnesiumlegierung mit 10 Gew.-% Dy und 1 Gew.-% Nd.

Figur 2: zeigt eine Aufnahme zur Bestimmung der Korngröße einer Magnesiumlegierung mit 10 Gew.-% Dy,1 Gew.-% Nd und 0,6 Gew.-% Zr.

Figur 3: zeigt eine Aufnahme zur Bestimmung der Korngröße einer Magnesiumlegierung mit 10 Gew.-% Dy 1 Gew.-% Nd und 0,2 Gew.-% Zr. Die Korngröße liegt bei 102 $\mu$m.

Figur 4: zeigt eine Aufnahme zur Bestimmung der Korngröße einer Magnesiumlegierung mit 10 Gew.-% Dy 1 Gew.-% Nd und 0,4 Gew.-% Zr. Die Korngröße liegt bei 68 $\mu$m.

Figur 5: zeigt eine Aufnahme zur Bestimmung der Korngröße einer Magnesiumlegierung mit 10 Gew.-% Dy 1 Gew.-% Nd und 0,6 Gew.-% Zr. Die Korngröße liegt bei 64 $\mu$m.

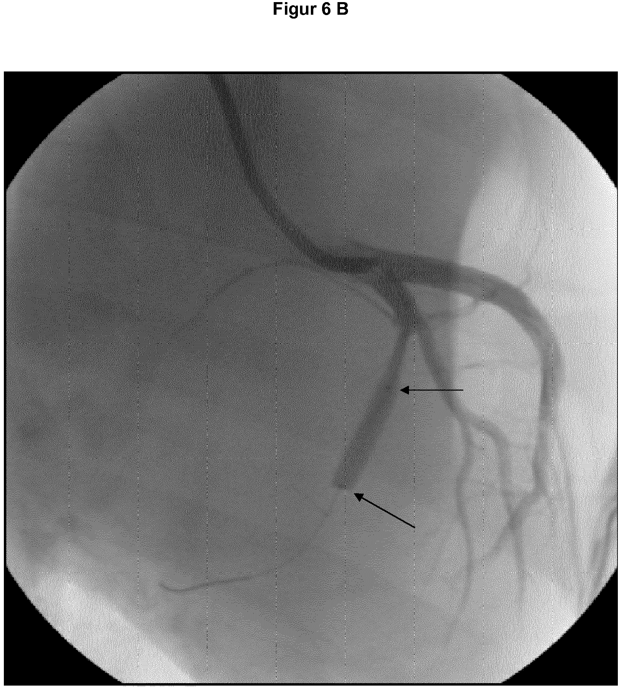

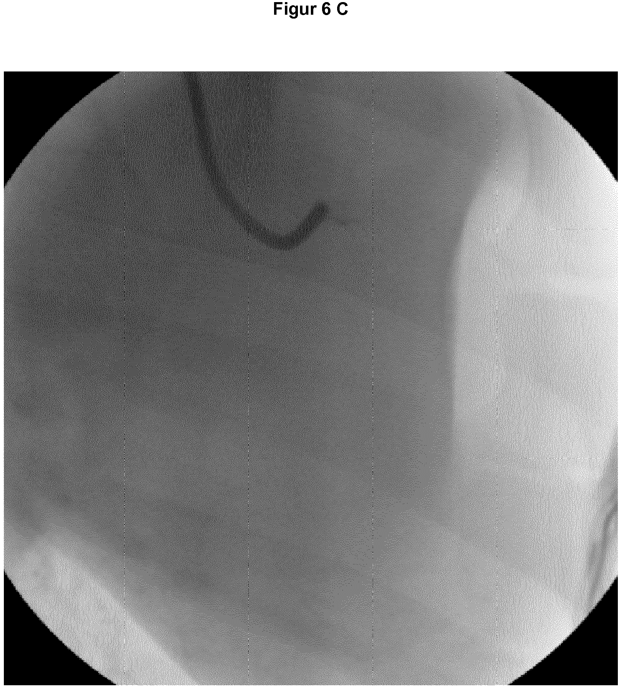

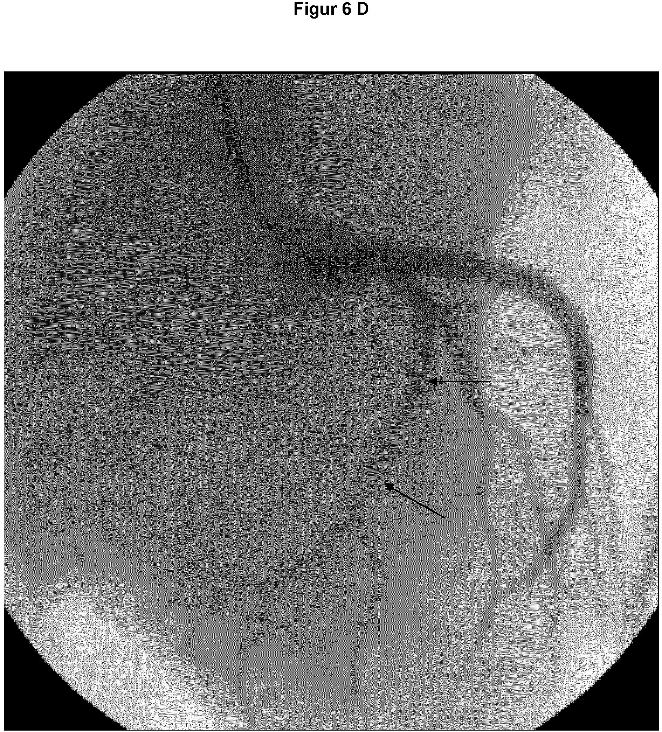

Figur 6: zeigt 4 Momentaufnahmen während der Ballonangioplastie zur Implantationen eines unbeschichteten, erfindungsgemäßen Stents an einem Schwein (s. Beispiel 8). Figur 6A wurde ohne Röntgenkontrastmittel aufgenommen, nachdem der Katheter bis zur entsprechenden Koronararterie vorgeschoben wurde. Die beiden Pfeile weisen auf die röntgendichten Markierungen distal und proximal des noch gefalteten Katheterballons auf dem der Stent angebracht ist. Figur 6B wurde mit Röntgenkontrastmittel während der Dilatation des Katheterballons um den Stent auf zu weiten und zu platzieren aufgenommen. Die beiden Pfeile markieren die Enden des Katheterballons. Der Katheterballon verschließt das Gefäß, so dass das Röntgenkontrastmittel nicht in den dahinterliegenden Teil des Gefäßes vordringen kann. Das Verhältnis Stent-Ballon zu Arterie lag bei 1,2 zu 1. Figur 6C wurde ohne Röntgenkontrastmittel aufgenommen nachdem der Katheter mit dem Ballon wieder aus dem Gefäß zurückgezogen wurde. Der Stent verbleibt dabei in dem Gefäß, ist aber auf der Aufnahme nicht zu sehen, da die Magnesiumlegierung nicht röntgendicht ist.

Figur 6D wurde mit Röntgenkontrastmittel aufgenommen nachdem der Katheter mit dem Ballon wieder aus dem Gefäß zurückgezogen wurde. Der Stent verbleibt dabei in dem Gefäß. Die Pfeile weisen auf die Enden des Stents. Im Bereich des Stents sammelt sich etwas mehr des Kontrastmittels. Der Stent selbst ist aber nicht zu erkennen.

Figur 7: zeigt eine graphische Darstellung der Ergebnisse der Korrosionsversuche mit binären Magnesiumlegierungen enthaltend zwischen 5 und 20 % Dysprosium und den Rest Magnesium. Gemessen wurde die Korrosion in 0,9%iger Kochsalzlösung im Eudiometer. Die Angaben in % beziehen sich auf den Anteil an Dysprosium in Gew.-%.

Figur 8: zeigt eine graphische Darstellung der Anhängigkeit der Tendenz zur Heißrissbildung im Zusammenhang der Menge an Zink in der Legierung. Getestet wurden Magnesiumlegierungen enthaltend 10% Dysprosium, 1,0 Gew.-% Neodym, steigende Gew.-% Zink, 0,2 Gew.-% Zirkonium und den Rest Magnesium. Die Angaben in % beziehen sich auf den Anteil an Zink in Gew.-%.

**Beispiele**

**Beispiel 1: Herstellung der Legierungen**

Die Legierungen wurden im sogenannten "Tütengußverfahren" hergestellt. Dieses Verfahren dient der Herstellung von Vormaterial für das spätere Strangpressen und zeichnet sich dadurch aus, dass das Material mit homogener Mikrostruktur und einer homogen Verteilung von Legierungselementen im Gußblock hergestellt werden kann. Damit eignet es sich außerordentlich dafür, kleinere Mengen hochwertige Bolzen für die Umformung herzustellen.
Bei diesem Verfahren werden die Magnesiumlegierungen (L1, L2, ... L34) in einem geschlichteten Stahltiegel erschmolzen. Als Tiegelwerkstoff kann praktisch jeder nickelfreie Stahl verwendet werden. Graphit wäre eine weitere Möglichkeit. Alle Schmelzoperationen finden unter Schutzgas statt. Die Schmelzbadtemperaturen liegen dabei im Bereich von 660-740°C. Bei Erreichen der Schmelzbadtemperatur wurden die Legierungselemente in Form von reinen Elementen oder als Masterlegierungen zugegeben. Nach der Zugabe der Legierungselemente wurde die Schmelze mechanisch gerührt. Die Rührzeit ist davon abhängig, wie lange die Elemente oder Masterlegierungen brauchen, um sich vollständig in der Schmelze aufzulösen. Nach dieser Vorbereitung wurde die Schmelze in eine dünnwandige Kokille überführt, die auf eine Temperatur von 600°C vorgeheizt wurde. Nach einer Dauer von ca. 60 Minuten wurde die Kokille in ein Wasserbad eingetaucht, das eine Temperatur von 15-20°C aufwies. Durch das Eintauchen erstarrte die Kokille komplett. Vor dem Strangpressen wurde die Oberfläche des Gußteils auf den Durchmesser des Rezipienten der Strangpresse abgedreht. Zudem wurde vor dem Strangpressen der Bolzen auf eine Temperatur von 250-500°C aufgeheizt und für 3 - 6 Stunden bei dieser Temperatur gehalten, um intermetallische Phasen aufzulösen bzw. Seigerungen zu homogenisieren. Im Anschluß daran erfolgte das Strangpressen und der so hergestellte Strang wurde an der Luft bis auf Raumtemperatur abgekühlt. Es wurden Drähte erhalten, welche danach in Rohre umgeformt wurden.
Die folgenden Legierungen wurden hergestellt:
Legierung L1:

| | |
|---|---|
| 87,8 Gew.-% | Magnesium |
| 10,0 Gew.-% | Dysprosium |
| 1,0 Gew.-% | Neodym |
| 1,0 Gew.-% | Zink |
| 0,2 Gew.-% | Verunreinigungen umfassend Si, Ni, Fe, Cu sowie weitere Metalle und Nichtmetalle. |

Legierung L2:

| | |
|---|---|
| 88,6 Gew.-% | Magnesium |
| 10,0 Gew.-% | Dysprosium |
| 1,0 Gew.-% | Neodym |
| 0,2 Gew.-% | Zirkonium |
| 0,2 Gew.-% | Verunreinigungen umfassend Si, Ni, Fe, Cu sowie weitere Metalle und Nichtmetalle. |

Legierung L3:

| | |
|---|---|
| 87,6 Gew.-% | Magnesium |
| 10,0 Gew.-% | Dysprosium |
| 1,0 Gew.-% | Neodym |
| 1,0 Gew.-% | Zink |
| 0,2 Gew.-% | Zirkonium |

(fortgesetzt)

| | |
|---|---|
| 0,2 Gew.-% | Verunreinigungen umfassend Si, Ni, Fe, Cu sowie weitere Metalle und Nichtmetalle. |

Legierung L4:

| | |
|---|---|
| 89,7 Gew.-% | Magnesium |
| 6,0 Gew.-% | Dysprosium |
| 2,0 Gew.-% | Neodym |
| 2,0 Gew.-% | Zink |
| 0,3 Gew.-% | Verunreinigungen umfassend Si, Ni, Fe, Cu sowie weitere Metalle und Nichtmetalle. |

Legierung L5:

| | |
|---|---|
| 90,7 Gew.-% | Magnesium |
| 5,5 Gew.-% | Dysprosium |
| 3.0 Gew.-% | Neodym |
| 0,5 Gew.-% | Zirkonium |
| 0,3 Gew.-% | Verunreinigungen umfassend Si, Ni, Fe, Cu sowie weitere Metalle und Nichtmetalle. |

Legierung L6:

| | |
|---|---|
| 87,4 Gew.-% | Magnesium |
| 8,0 Gew.-% | Dysprosium |
| 2,2 Gew.-% | Neodym |
| 1,8 Gew.-% | Zink |
| 0,3 Gew.-% | Zirkonium |
| 0,3 Gew.-% | Verunreinigungen umfassend Si, Ni, Fe, Cu sowie weitere Metalle und Nichtmetalle. |

Legierung L7:

| | |
|---|---|
| 82,7 Gew.-% | Magnesium |
| 12,0 Gew.-% | Dysprosium |
| 2,5 Gew.-% | Neodym |
| 2,5 Gew.-% | Zink |
| 0,3 Gew.-% | Verunreinigungen umfassend Si, Ni, Fe, Cu sowie weitere Metalle und Nichtmetalle. |

Legierung L8:

| | |
|---|---|
| 74,2 Gew.-% | Magnesium |
| 22,5 Gew.-% | Dysprosium |
| 2,6 Gew.-% | Neodym |
| 0,4 Gew.-% | Zirkonium |
| 0,3 Gew.-% | Verunreinigungen umfassend Si, Ni, Fe, Cu sowie weitere Metalle und Nichtmetalle. |

Legierung L9:

| | |
|---|---|
| 83,1 Gew.-% | Magnesium |
| 15,2 Gew.-% | Dysprosium |
| 1,2 Gew.-% | Neodym |
| 0,2 Gew.-% | Zirkonium |
| 0,3 Gew.-% | Verunreinigungen umfassend Si, Ni, Fe, Cu sowie weitere Metalle und Nichtmetalle. |

Legierung L10:

| | |
|---|---|
| 88,9 Gew.-% | Magnesium |
| 8,0 Gew.-% | Dysprosium |
| 1,4 Gew.-% | Neodym |
| 1,2 Gew.-% | Zink |
| 0,2 Gew.-% | Zirkonium |
| 0,3 Gew.-% | Verunreinigungen umfassend Si, Ni, Fe, Cu sowie weitere Metalle und Nichtmetalle. |

Legierung L11:

| | |
|---|---|
| 90,6 Gew.-% | Magnesium |
| 8,0 Gew.-% | Dysprosium |
| 1,0 Gew.-% | Neodym |
| 0,2 Gew.-% | Zink |
| 0,2 Gew.-% | Zirkonium |

Legierung L12:

| | |
|---|---|
| 89,3 Gew.-% | Magnesium |
| 8,0 Gew.-% | Dysprosium |
| 1,0 Gew.-% | Neodym |
| 1,0 Gew.-% | Europium |
| 0,5 Gew.-% | Zink |
| 0,2 Gew.-% | Zirkonium |

Legierung L13:

| | |
|---|---|
| 86,0 Gew.-% | Magnesium |
| 12,0 Gew.-% | Dysprosium |
| 1,0 Gew.-% | Neodym |
| 0,8 Gew.-% | Zink |
| 0,2 Gew.-% | Zirkonium |

Legierung L14:

| | |
|---|---|
| 90,1 Gew.-% | Magnesium |
| 6,0 Gew.-% | Dysprosium |
| 1,0 Gew.-% | Neodym |
| 1,0 Gew.-% | Europium |
| 1,5 Gew.-% | Zink |
| 0,4 Gew.-% | Zirkonium |

Legierung L15:

| | |
|---|---|
| 86,8 Gew.-% | Magnesium |
| 10,0 Gew.-% | Dysprosium |
| 1,0 Gew.-% | Neodym |
| 1,0 Gew.-% | Europium |
| 1,0 Gew.-% | Zink |
| 0,2 Gew.-% | Zirkonium |

Legierung L16:

| | |
|---|---|
| 82,8 Gew.-% | Magnesium |
| 14,0 Gew.-% | Dysprosium |
| 0,5 Gew.-% | Neodym |
| 0,5 Gew.-% | Europium |
| 2,0 Gew.-% | Zink |
| 0,2 Gew.-% | Zirkonium |

Legierung L17:

| | |
|---|---|
| 87,3 Gew.-% | Magnesium |
| 10,0 Gew.-% | Dysprosium |
| 1,5 Gew.-% | Neodym |
| 1,0 Gew.-% | Zink |
| 0,2 Gew.-% | Zirkonium |

Legierung L18:

| | |
|---|---|
| 87,45 Gew.-% | Magnesium |
| 10,0 Gew.-% | Dysprosium |
| 1,5 Gew.-% | Neodym |
| 1,0 Gew.-% | Zink |
| 0,05 Gew.-% | Eisen |

Legierung L19:

| | |
|---|---|
| 83,1 Gew.-% | Magnesium |
| 15,0 Gew.-% | Dysprosium |
| 0,9 Gew.-% | Neodym |
| 1,0 Gew.-% | Zirkonium |

Legierung L20:

| | |
|---|---|
| 95,0 Gew.-% | Magnesium |
| 4,5 Gew.-% | Dysprosium |
| 0,5 Gew.-% | Neodym |

Legierung L21:

| | |
|---|---|
| 73,7 Gew.-% | Magnesium |
| 20,0 Gew.-% | Dysprosium |
| 5,0 Gew.-% | Neodym |
| 1,0 Gew.-% | Zink |
| 0,3 Gew.-% | Zirkonium |

Legierung L22:

| | |
|---|---|
| 87,25 Gew.-% | Magnesium |
| 10,0 Gew.-% | Dysprosium |
| 1,5 Gew.-% | Neodym |

(fortgesetzt)

| | |
|---|---|
| 1,0 Gew.-% | Zink |
| 0,05 Gew.-% | Eisen |
| 0,2 Gew.-% | Zirkonium |

Legierung L23:

| | |
|---|---|
| 85,8 Gew.-% | Magnesium |
| 12,0 Gew.-% | Dysprosium |
| 1,0 Gew.-% | Neodym |
| 1,0 Gew.-% | Zink |
| 0,2 Gew.-% | Zirkonium |

Legierung L24:

| | |
|---|---|
| 82,1 Gew.-% | Magnesium |
| 15,0 Gew.-% | Dysprosium |
| 0,9 Gew.-% | Neodym |
| 1,0 Gew.-% | Zink |
| 1,0 Gew.-% | Zirkonium |

Legierung L25:

| | |
|---|---|
| 79,1 Gew.-% | Magnesium |
| 20,0 Gew.-% | Yttrium |
| 0,9 Gew.-% | Europium |

Legierung L26:

| | |
|---|---|
| 92,5 Gew.-% | Magnesium |
| 5,0 Gew.-% | Dysprosium |
| 2,5 Gew.-% | Europium |

Legierung L27:

| | |
|---|---|
| 82,1 Gew.-% | Magnesium |
| 15,5 Gew.-% | Dysprosium |
| 1,2 Gew.-% | Neodym |
| 1,0 Gew.-% | Zink |
| 0,2 Gew.-% | Zirkonium |
| 0,001 Gew.-% | Verunreinigungen umfassend Si, Ni, Fe, Cu sowie weitere Metalle und Nichtmetalle. |

Legierung L28:

| | |
|---|---|
| 72,0 Gew.-% | Magnesium |
| 20,0 Gew.-% | Gadolinium |
| 5,0 Gew.-% | Neodym |
| 1,0 Gew.-% | Zink |
| 2,0 Gew.-% | Zirkonium |

Legierung L29:

|  |  |
|---|---|
| 88,8 Gew.-% | Magnesium |
| 6,0 Gew.-% | Dysprosium |
| 4,0 Gew.-% | Europium |
| 1,0 Gew.-% | Zink |
| 0,2 Gew.-% | Zirkonium |

Legierung L30:

|  |  |
|---|---|
| 89,8 Gew.-% | Magnesium |
| 8,0 Gew.-% | Dysprosium |
| 1,0 Gew.-% | Europium |
| 1,0 Gew.-% | Zink |
| 0,2 Gew.-% | Zirkonium |

Legierung L31:

|  |  |
|---|---|
| 73,2 Gew.-% | Magnesium |
| 25,0 Gew.-% | Dysprosium |
| 0,4 Gew.-% | Neodym |
| 1,4 Gew.-% | Europium |

Legierung L32:

|  |  |
|---|---|
| 87,4 Gew.-% | Magnesium |
| 10,0 Gew.-% | Dysprosium |
| 1,0 Gew.-% | Europium |
| 0,5 Gew.-% | Neodym |
| 1,0 Gew.-% | Zink |
| 0,1 Gew.-% | Zirkonium |

Legierung L33:

|  |  |
|---|---|
| 87,0 Gew.-% | Magnesium |
| 10,0 Gew.-% | Dysprosium |
| 0,3 Gew.-% | Europium |
| 1,5 Gew.-% | Neodym |
| 1,0 Gew.-% | Zink |
| 0,2 Gew.-% | Zirkonium |

Legierung L34:

|  |  |
|---|---|
| 86,0 Gew.-% | Magnesium |
| 12,0 Gew.-% | Dysprosium |
| 1,0 Gew.-% | Europium |
| 0,8 Gew.-% | Zink |
| 0,2 Gew.-% | Zirkonium |

**Beispiel 2: Rohrherstellung**

Aus den Legierungen L1 bis L10 gemäß Beispiel 1 wurden stranggepresste Drähte hergestellt. In diesen stranggepressten Drähten wird eine Präzisionsbohrung in Längsrichtung eingebracht, welche bereits die Wandstärke der späteren Stents mitbestimmt. Durch mehrere Umformschritte wird ein Rohr mit vorgegebenem Durchmesser und bestimmter Wandstärke hergestellt. Zwischen den einzelnen Umformschritten findet eine wiederkehrende Wärmebehandlung statt.

**Beispiel 3: Stentfertigung**

Ein gemäß Beispiel 2 hergestelltes Rohr wird in eine Aufnahme in der Lasermaschine fixiert. Ein gepulster Festkörperlaser (FKL) schneidet aus dem Rohr die Konturen des Stentdesigns heraus. Das Laserschneiden erfolgt unter Schutzgasatmosphäre.

Das Stentdesign ist in einem NC-Programm (numerical control = Numerische Steuerung) hinterlegt. Dieses gibt dem Laser die Verfahrwege vor, nach denen das Rohr strukturiert wird. Durch das Laserstrahlschneiden kommt es zur Gradbildung, insbesondere auf der Rohrinnenseite, entlang der gesamten Schneidkontur. Dies kann dazu führen, daß Reststücke und Ausschnitte nach Beendigung des Schneidvorgangs in der Kontur haften bleiben. Die Reststücke und Ausschnitte werden mechanisch entfernt und der Stent von Fertigungsrückständen gereinigt. In einer ersten optischen Sichtkontrolle wird eine Inspektion der Schneidkontur durchgeführt.

Im Folgenden wird der Stent elektro-chemisch poliert. Der Stent wird anodisch beschaltet und in ein Säurebad getaucht. Über eine im Bad fixierte Kathode wird ein Stromkreis geschlossen. Der Stromkreis bleibt für mehrere Minuten aufrechterhalten. Die Elektropolitur ist ein umgekehrter galvanischer Prozeß bei dem kontrolliert Material von der Oberfläche des anodisch beschalteten Bauteils abgetragen wird. Dabei wird durch das Verfahren bedingt bevorzugt an scharf kantigen Ecken und Kanten abgetragen. Der Stent erhält eine glatte Oberfläche und abgerundete Kanten entlang der Konturen. Nach der Politur wird der Stent gereinigt und von Säurerückständen befreit. In der Endreinigung werden alle noch verbliebenden Fertigungsrückstände von der Stentoberfläche entfernt. In einer letzen optischen Sichtkontrolle wird die Stentgeometrie vermessen und die Oberfläche auf Reinheit geprüft.

**Beispiel 4: Bestimmung der Korngröße**

Die Auszählung der Korngröße wurde im Linienschnittverfahren vorgenommen. Körner die am Ende der Geraden nur halb geschnitten sind wurden hierbei als halbe Körner gezählt. Die Vergrößerung wurde so gewählt, dass durch das Linienraster mindestens 50 Körner geschnitten wurden. Es wurden mindestens 5 Stellen, mit einer Gesamtanzahl von mindestens 250 Schnittpunkten, auf der Probe ausgewertet.

**Beispiel 5: Bestimmung der Korrosionsrate**

Bei Raumtemperatur wurden über einen Zeitraum von 3 Tagen die Korrosionsraten verschiedener Legierungen in physiologischer Kochsalzlösung bestimmt (s. Tabelle 1). Getestet wurde eine Legierung enthaltend 90,8 Gew.-% Mg, 8 Gew.-% Dy, 1 Gew.-% Nd und 0,2 Gew.-% Zr, eine Legierung enthaltend 89,8 Gew.-% Mg, 8 Gew.-% Dy, 1 Gew.-% Nd, 1 Gew.-% Eu und 0,2 Gew.-% Zr, eine Legierung enthaltend 86,8 Gew.-% Mg, 12 Gew.-% Dy, 1 Gew.-% Nd und 0,2 Gew.-% Zr und eine Legierung enthaltend 87,8 Gew.-% Mg, 10 Gew.-% Dy, 1 Gew.-% Nd, 1 Gew.-% Eu und 0,2 Gew.-% Zr. Zusätzlich wurden Legierungen getestet enthaltend 1,0 Gew.-% Neodym, 1,0 Gew.-%Zink, 0,2 Gew.-% Zirkonium, zwischen 5 und 20 % Dysprosium und den Rest Magnesium (s. Abbildung 7). Korrosionsprodukte wurden durch Eintauchen der Proben in Chromsäure (180 g/L) für 20 min bei Raumtemperatur entfernt. Die durchschnittliche Korrosionsrate wurde in Millimeter pro Jahr mit Hilfe der folgenden Gleichung berechnet:

$$CR = \frac{8.76 \times 10^4 \times \Delta\,\text{g}}{\text{A} \cdot t \cdot \rho}$$

Tabelle 1: Korrosionsrate der Legierungen, gemessen über 3 Tage bei Raumtemperatur und in 0,9% NaCl; die Angaben zu den Komponenten der Legierungen sind in Gew.-% und Mg ist als Hauptkomponente füllt immer bis auf 100% der Legierung auf. Getestet wurden die Legierungen nach dem Guß ohne Wärmebehandlung angegeben sind die Mittelwerte und Standardabweichungen der unterschiedlichen Legierungen.

| Nr. | Zusammensetzung | Korrosionsrate (mm/Jahr) |
|---|---|---|
| L11 | Mg8Dy1Nd0,2Zn0,2Zr | 9,25 ±0,38 |
| L15 | Mg10Dy1Nd1Eu1Zn0,2Zr | 0,81±0,06 |
| L23 | Mg12Dy1 Nd1 Zn0,2Zr | 2,94±1,88 |

(fortgesetzt)

| Nr. | Zusammensetzung | Korrosionsrate (mm/Jahr) |
|---|---|---|
| L16 | Mg8Dy1 Nd1 Eu1Zn0,1Zr | 4,9±1,62 |
| L14 | Mg6Dy1 Nd1 Eu1,5Zn0,4Zr | 9,56±0,29 |
| L16 | Mg14Dy0,5Nd0,5Eu2Zn0,2Zr | 1,25±0,12 |
| L18 | Mg10Dy1,5Nd1Zn0,05Fe | 12,41±2,16 |
| L20 | Mg4,5Dy0,5Nd | 25,56±2,34 |
| L24 | Mg15Dy0,9Nd1Zr1Zn | 2,98±1,78 |
| L25 | Mg20Y0,9Eu | 44,71±3,22 |
| L28 | Mg20Gd5Nd1Zn2Zr | 38,96±1,34 |
| L30 | Mg8Dy1Eu1Zn0,2Zr | 3,88±1,87 |
| L22 | Mg10Dy1,5Nd1Zn0,2Zr0,05Fe | 4,47±2,11 |
| L34 | Mg12Dy1Eu0,8Zn0,2Zr | 5,46±1,22 |
| L29 | Mg6Dy4Eu1Zn0,2Zr | 12,20±1,36 |
| L33 | Mg10Dy0,3Eu1,5Nd1Zn0,2Zr | 1,25±0,67 |
| L26 | Mg5Dy2,5Eu | 23.56±1,56 |
| L31 | Mg25Dy0,4Nd1,4Eu | 48,71±1,87 |

**Beispiel 6: Mechanische Kennwerte der Legierungen**

Die Legierungen und Gußteile wurden hergestellt, wie im Beispiel 1 beschrieben und straggepresst. Die Wärmebehandlung T4 erfolgte bei 510°C über 8h und eventuell anschließend die Wärmebehandlung T6 bei 200°C über einen Zeitraum von 72h. Nach der T4-Wärmebenadlung wurden die Proben unmittelbar in Wasser abgeschreckt. Alle Proben wurden von der gleichen Position der Blöcke entnommen.

Die Zugversuche wurden bei Raumtemperatur gemäß DIN EN 10002-1 (entspricht ISO 6892 und ASTM E8) und Druckversuche wurden bei Raumtemperatur gemäß DIN 50106 (entspricht ISO 604 und ASTM D695) durchgeführt. Es wurden mindestens 3 Proben für jeden Wert getestet. Die Zugfestigkeit wurde aus der maximal erreichten Zugkraft im Zugversuch bezogen auf den ursprünglichen Querschnitt der Probe berechnet.

EP 2 744 531 B1

Tabelle 2: Mechanische Kennwerte Legierungen. Getestet wurden die Legierungen als Probe nach dem Strangpressen (ST, ohne Wärmebehandlung) und nach unterschiedlicher Wärmebehandlung, T4 (lösungsgeglüht) und T6(eine weitere Wärmebehandlung nach T4, auch "Auslagern" genannt). Die Angaben zu den Komponenten der Legierungen sind in Gew.-% und Mg als Hauptkomponente ergänzt die Mengenangabe immer bis auf 100% der Legierung. SD steht für die Standardabweichung der Mittelwerte, die in der Spalte zuvor angegeben sind (n=3).

| | Zusammensetzung | Streckgrenze (MPa) | SD | Zugfestigkeit (MPa) | SD | Bruchdehnung (%) | SD |
|---|---|---|---|---|---|---|---|
| ST | Mg8Dy1Nd0,2Zn0,2Zr | 107,33 | 1,8 | 208,5 | 0,85 | 28,12 | 3,41 |
| T4 | | 87,54 | 0,46 | 176,84 | 2,03 | 18,83 | 1,79 |
| T6 | | 97,95 | 1,67 | 194,11 | 1,1 | 19,33 | 0,68 |
| ST | Mg10Dy1Nd1Eu1Zn0,2Zr | 169,30 | 0,74 | 283,89 | 0,68 | 16,96 | 1 |
| T4 | | 151,97 | 1,77 | 259,50 | 2,57 | 18,02 | 0,29 |
| T6 | | 159,23 | 2,23 | 275,55 | 1,78 | 18,15 | 2,77 |
| ST | Mg12Dy1Nd1Zn0,2Zr | 126,07 | 1,8 | 226,04 | 0,35 | 28,55 | 0,08 |
| T4 | | 98,38 | 0,43 | 188,45 | 0,5 | 20,47 | 0,91 |
| T6 | | 114,6 | 1,69 | 205,2 | 1,25 | 17,99 | 0,79 |
| ST | Mg8Dy1Nd1 Eu1Zn0,1Zr | 132,24 | 1,1 | 227,21 | 0,59 | 19,75 | 1,11 |
| T4 | | 114,93 | 1,25 | 210,73 | 1,51 | 20,89 | 1,01 |
| T6 | | 136,77 | 1,77 | 223,28 | 0,67 | 23,64 | 2,01 |
| ST | Ma6Dy1Nd1Eu1,5Zn0,4Zr | 128,14 | 8,02 | 202,74 | 2,91 | 24,62 | 2,09 |
| T4 | | 80,97 | 2,27 | 173,47 | 2,02 | 23,78 | 3,52 |
| T6 | | 84,26 | 2,57 | 178,26 | 1,35 | 26,32 | 2,5 |
| ST | Mg14Dy0,5Nd0,5Eu2Zn0,2Zr | 165,64 | 4,95 | 218,17 | 3,07 | 18,9 | 1,14 |
| T4 | | 110,78 | 1,87 | 201,28 | 1,19 | 21,62 | 1,07 |
| T6 | | 153,15 | 3,55 | 264,09 | 0,71 | 17,66 | 1,33 |
| ST | Mg10Dv1,5Nd1Zn0,05Fe | 145,46 | 3,55 | 237,21 | 0,75 | 28,9 | 1,73 |
| T4 | | 102,78 | 4,38 | 193,36 | 5,84 | 27,57 | 0,88 |
| T6 | | 108,84 | 1,68 | 200,16 | 2,97 | 25,56 | 1,66 |
| ST | Mg4,5Dy0,5Nd | 68,39 | 7,9 | 208,48 | 2,03 | 28,4 | 0,72 |
| T4 | | 60,31 | 1,71 | 179,04 | 0,83 | 23,17 | 0,38 |

(fortgesetzt)

| | Zusammensetzung | Streckgrenze (MPa) | SD | Zugfestigkeit (MPa) | SD | Bruchdehnung (%) | SD |
|---|---|---|---|---|---|---|---|
| T6 | | 75,13 | 1,32 | 250,34 | 1,42 | 13,34 | 0,74 |
| ST | Mg15Dy0,9Nd1Zr1Zn | 136,93 | 1,6 | 227,07 | 0,42 | 22,9 | 3,03 |
| T4 | | 95,79 | 1,94 | 200,59 | 2,59 | 21,57 | 0,34 |
| T6 | | 112,09 | 0,41 | 206,11 | 0,19 | 19,56 | 0,66 |
| ST | Mq20Y0,9Eu | 159,75 | 1,99 | 238,55 | 0,76 | 11,57 | 0,58 |
| T4 | | 123,19 | 4,83 | 214 | 1,42 | 19,62 | 2,74 |
| T6 | | 144,08 | 4,37 | 220,2 | 2,58 | 15,58 | 0,94 |
| ST | Mg20Gd5Nd1Zn2Zr | 297,75 | 8,12 | 338,53 | 5,67 | 1,53 | 0,27 |
| T4 | | 195,82 | 15,65 | 276,89 | 0,91 | 6,58 | 0,95 |
| T6 | | 327,07 | 17,57 | 378,45 | 14,94 | 0,76 | 0,32 |
| ST | Mg8Dy1Eu1Zn0,2Zr | 112,85 | 1,15 | 198,9 | 0,43 | 24,07 | 1,05 |
| T4 | | 93,5 | 1,01 | 182,38 | 0,91 | 24,02 | 0,81 |
| T6 | | 99 | 0,99 | 185,7 | 0,4 | 25,9 | 1,16 |
| ST | Mg10Dy1,5Nd1Zn0,2Zr0,05Fe | 127,8 | 4,62 | 215,84 | 1 | 19,39 | 1,4 |
| T4 | | 96,72 | 4,02 | 192,99 | 2,87 | 25,92 | 0,98 |
| T6 | | 112,34 | 3,1 | 201,35 | 2,18 | 24,44 | 1,91 |
| ST | Mg12Dy1Eu0,8Zn0,2Zr | 182,30 | 1,52 | 293,62 | 1,37 | 22,39 | 2,06 |
| T4 | | 164,48 | 1,44 | 268,66 | 0,45 | 23,70 | 1,63 |
| T6 | | 172,34 | 2,12 | 271,35 | 1,82 | 23,34 | 1,79 |
| ST | Mg6Dy4Eu1Zn0,2Zr | 115,09 | 1,39 | 208,3 | 1,68 | 2,30 | 0,51 |
| T4 | | 97,55 | 0,74 | 189,39 | 0,84 | 4,78 | 1,71 |
| T6 | | 112,58 | 1,59 | 196,71 | 2,31 | 3,41 | 0,69 |
| ST | Mg10Dy0,3Eu1,5Nd1Zn0,2Zr | 168,54 | 6,15 | 277,11 | 2,09 | 16,46 | 2,33 |
| T4 | | 136,36 | 5,11 | 244,89 | 2,37 | 20,67 | 3,15 |
| T6 | | 152,22 | 2,42 | 253,91 | 2,33 | 18,56 | 1,87 |

(fortgesetzt)

| | Zusammensetzung | Streckgrenze (MPa) | SD | Zugfestigkeit (MPa) | SD | Bruchdehnung (%) | SD |
|---|---|---|---|---|---|---|---|
| ST | Mg5Dy2,5Eu | 74,25 | 1,63 | 283,50 | 1,44 | 21,60 | 1,27 |
| T4 | | 60,19 | 1,69 | 264,46 | 0,91 | 23,16 | 1,43 |
| T6 | | 65,38 | 1,83 | 266,64 | 1,36 | 22,85 | 1,64 |
| ST | Mg25Dy0,4Nd1,4Eu | 106,34 | 2,98 | 211,15 | 1,65 | 18,90 | 1,55 |
| T4 | | 88,74 | 1,69 | 178,56 | 2,03 | 20,03 | 2,31 |
| T6 | | 94,21 | 1,34 | 191,25 | 1,67 | 19,54 | 1,99 |

**Beispiel 7: Tierversuchsstudie**

8 Stents hergestellt gemäß Beispiel 2 und 3 wurden in die Koronarien von 4 Hausschweinen implantiert. Die Stents hatten einen Durchmesser von 3,0 mm und eine Länge von 14 mm (Länge des Katheterballons 15 mm), waren nicht beschichtet (BMS) und waren aus einer Legierung der folgenden Zusammensetzung hergestellt:

|  |  |
|---|---|
| 87,8 Gew.-% | Magnesium |
| 10,0 Gew.-% | Dysprosium |
| 1,0 Gew.-% | Neodym |
| 1,0 Gew.-% | Zink |
| 0,2 Gew.-% | Zirkonium |

Der gewählte "follow up" Zeitpunkt für alle 4 Tiere war 4 Wochen nach Implantation. Einen Tag vor der Stentimplantation wurde den Schweinen eine Einzeldosis Clopidogrel (300mg) und Aspirin (250mg) oral verabreicht. Unter Vollnarkose wurde chirurgisch ein Zugang zu der Femoralarterie gelegt und einem Bolus von Natrium-Heparin (10 000 IU) verabreicht. Ein 6F koronarer Führungskatheter wurde durch Femoralarterie in die Aorta descendens eingeführt. Die Koronarangiographie wurde durch eine Injektion nicht-ionischen Kontrastmittels per Hand durchgeführt, um die anatomischen Gegebenheiten für die Durchführung des Verfahrens zu erhalten.

Die Stents wurden im *Ramus interventricularis anterior* (RIVA oder engl. LAD) und *Ramus circumflexus* (RCX oder engl. LCx) implantiert. Der Dilatationsdruck des Ballons zur Implantation des Stents wurde so gewählt, dass eine Stent-Ballon zu Arterie Verhältnis von 1,2 zu 1 erreicht wurde. Die Schweine bekamen die Möglichkeit sich anschließend zu erholen. Während der gesamten 4 Wochen "follow up" wurde den Tieren täglich 100mg Aspirin und 75mg Clopidogrel pro 30g Körpergewicht oral verabreicht.

Nach 4 Wochen "follow up", wurden die Kontrollangiographie und die optische Kohärenztomographie (OCT) durchgeführt.

Im Rahmen des OCT Verfahren wurde ein 0,014 Inch Führungsdraht wird in den RIVA und den RCX eingefügt und durch die implantierten Stents in den distalen Teil des Gefäßes geführt. Ein intravaskulärer OCT-Katheter wurde anschließend über den Führungsdraht bis distal des Stent vorgeschoben. Die Injektionspumpe wurde eingeschaltet, um Kontrastmittel mit einer Geschwindigkeit von 3,0 ml/s zu injizieren und somit das Blut vorübergehend zu verdrängen. Die gesamte Länge der Läsion wird mit Hilfe einer automatischen Rückzugshilfe und 10 mm/s abgebildet. Nach den Aufnahmen wird der OCT-Katheter zurückgezogen, und die Bilder gespeichert. Die Tiere wurden dann euthanasiert, und die Koronararterien explantiert.

Die explantierten Arterien wurden mit einem Druck von 100 mmHg für 1 h mit 7% Formalin perfusionsfixiert. Die Stents wurden für die Lichtmikroskopie verarbeitet. Die Arterien wurden für die Lichtmikroskopie in drei Teile geschnitten: proximale, mittlere und distale Stent-Segmente. Die gestenteten Abschnitte wurden in Methyl-metacrylat (Technovit 9100) eingebettet. Von den Segmenten der gestenteten Arterien werden unter Verwendung eines Rotationsmikrotoms 4-6 $\mu$m Schnitte angefertigt und mit Hämatoxylin und Eosin angefärbt.

Im Rahmen der Analyse wurden Details der Studie aufgelistet, wie die Stentposition, der Dilatationsdruck und die Dilatationszeit, sowie alle Komplikationen während der Implantation.

*Quantitative Koronarangioplastie (QCA)*

Eine QCA wurde zur Analyse der In-Stent-Restenose durchgeführt. Die folgenden Parameter wurden dabei bestimmt: Gefäßdurchmesser vor und nach Stentimplantation, den minimalen Lumendurchmesser (MLD) nach Stentimplantation und nach *follow up* und den Durchmesser eines Vergleichssegments nach *follow up*. Hierbei ist der minimale Lumendurchmesser der kleinste absolute Gefäßinnendurchmesser im Bereich des dilatierten Segments, gemittelt aus den zwei orthogonalen Projektionsebenen. LLL (late lumen loss) ist ein Mass der Verengung des Lumens durch neointimale Hyperplasie. Gemessen wird der Lumendurchmesser direkt nach der Intervention und 4 Wochen postinterventionell, die Differenz beider wird als LLL angegeben. Die Länge des stenosierten oder dilatierten Segments wurde kontrolliert und die prozentuale Stenose kalkuliert.

*Optische Kohärenztomographie (OCT)*

Die Bilder der optischen Kohärenztomographie wurden an Hand der relevanten Richtlinie (JACC, 2012) ausgewertet. Die folgenden Parameter wurden erhaben: Stent-Malapposition, Bedeckung der Stentstreben, Hervortreten des Gewebes, die arterielle Dissektion, Thrombose. Die quantitative Analyse der OCT-Bilder umfasst den minimalen und maximalen Stentdurchmesser und die Fläche des Lumens. Die folgenden Parameter wurden berechnet: maximale Stenoseausdehnung (area stenosis) und Stentsymmetrie. Für die quantitative Analyse wurde der "schlechteste" Querschnitt pro Testgruppe bestimmt.

Berechnung der Stenoseausdehnung (%AS):

%AS= Fläche der Intima/Stentfläche = (Stentfläche-Fläche des Lumens)/Stentfläche

Berechnung der Stentsymmetrie:

Stentsymmetrie = (Maximaler Stentdurchmesser-Minimaler Stentdurchmesser)/ Maximaler Stentdurchmesser

Fibrinablagerung, Entzündungsgrad (Intima und Adventitia), Einblutungen und Nekrose wurden analog zu publizierten Richtlinien ausgewertet.

*Histomorphometrie*

Die Histomorphometrie wurde mittels computer-assistierter Flächenmessung durchgeführt. Das Lumen, die Fläche der Membrana elastica interna und Membrana elastica externa und die maximale Dicke der Neointima wurden gemessen. Die Ausdehnung der Neointima und der Tunica media und die prozentuale Stenose wurden berechnet.

Ergebnisse

Der Dilatationsdruck der aufgewendet wurde lag zwischen 12 und 18 atm. Die Balloninflation dauerte 30 sec an. Generell war die Handhabung des Stents und Ballons exzellent, eine sehr gute Schubfähigkeit und sehr kurze Dauer der Deflation war zu verzeichnen.

Tabelle 3: Ergebnisse der Quantitativen Koronarangioplastie (QCA), angegeben sind die Mittelwerte und Standardabweichungen (SD); MLD=minimaler Lumendurchmesser, RD=Durchmesser eines Vergleichssegments, %DS=prozentualer Stenosedurchmesser, FUP=follow-up, LLL=Late Lumen Loss

| | Prä-MLD (mm) | Post-MLD (mm) | FUP-MLD (mm) | FUP-RD (mm) | FUP-%DS (%) | LLL (mm) |
|---|---|---|---|---|---|---|
| Unbeschichtete Stents (BMS) | 2,68 | 2,93 | 2,08 | 2,92 | 28,75 | 0,85 |
| SD | 0,11 | 0,07 | 0,53 | 0,20 | 16,79 | 0,47 |

Tabelle 4: Qualitative Analyse der Optischen Kohärenztomographie (OCT) je implantiertem Stent

| Tier-Nr. | Arterie | Gruppe | Stent-Malapposition | Hervortreten des Gewebes | In-Stent Thrombose | In-Stent Dissektion | Rand-Dissektion | Endothelialisierung |
|---|---|---|---|---|---|---|---|---|
| MEKO-1 | LAD | BMS | 0 | 0 | 0 | 0 | 0 | komplett |
| MEKO-1 | LCx | BMS | 0 | 0 | 0 | 0 | 0 | lückenhaft |
| MEKO-2 | LAD | BMS | 0 | 0 | 0 | 0 | 0 | komplett |
| MEKO-2 | LCx | BMS | 0 | 0 | 0 | 0 | 0 | komplett |
| MEKO-3 | LAD | BMS | 0 | 0 | 0 | 0 | 0 | komplett |
| MEKO-3 | LCx | BMS | 0 | 0 | 0 | 0 | 0 | komplett |
| MEKO-4 | LAD | BMS | 0 | 0 | 0 | 0 | 0 | komplett |
| MEKO-4 | LCx | BMS | 0 | 0 | 0 | 0 | 0 | komplett |

Tabelle 5: Qualitative Analyse der Optischen Kohärenztomographie (OCT) in Bezug zur Anzahl der implantierten Stents (n=8; alle Werte in Prozent)

| | Stent-Malapposition | Hervortreten des Gewebes | In-Stent Thrombose | In-Stent Dissektion | Rand-Dissektion | Abgeschlossene Endotheliali sierung |
|---|---|---|---|---|---|---|
| BMS n=8 | 0 | 0 | 0 | 0 | 0 | 87,5 |

Aus den Tabellen 3, 4 und 5 lässt sich entnehmen, dass zum einen bei Verwendung eines erfindungsgemäßen Stents keine der getesteten Komplikationen auftrat und zum anderen, dass eine Endothelialisierung nach 4 Wochen fast immer vollständig abgeschlossen war, was bedeutete, dass das erhöhte Risiko der In-Stent-Thrombose durch nicht abgeschlossene Endothelialisierung oder Endzündungsreaktionen nicht mehr vorliegt. Vergleichbare Ergebnisse wurden auch mit Stents aus einer Magnesiumlegierung enthaltend Europium an Stelle von Neodym erhalten.

Tabelle 6: weitere Ergebnisse der Qualitative Analyse der Optischen Kohärenztomographie (OCT), angegeben sind die Mittelwerte und Standardabweichungen (SD).

| Type | Min. Stentdurchmesser (mm) | max. Stentdurchmesser (mm) | Stentfläche (mm$^2$) | Lumenfläche (mm$^2$) | %AS (%) | Stentsymmetrie |
|---|---|---|---|---|---|---|
| BMS n=8 | 2,54 | 2,72 | 7,58 | 5,08 | 34,0 | 0,07 |
| SD | 0,34 | 0,35 | 1,80 | 1,69 | 13,2 | 0,02 |

**Patentansprüche**

1. Resorbierbarer Stent bestehend aus einer Magnesiumlegierung die bezogen auf das Gesamtgewicht der Legierung folgende Bestandteile enthält:

| | | | |
|---|---|---|---|
| 5,0 Gew.-% | - | 25,5 Gew.-% | Dysprosium |
| 0,01 Gew.-% | - | 5,0 Gew.-% | Neodym und/oder Europium. |
| 0,1 Gew.-% | - | 3,0 Gew.-% | Zink |
| 0,1 Gew.-% | - | 2,0 Gew.-% | Zirkonium |
| Rest bis | | 100,0 Gew.-% | Magnesium, |

wobei die Legierung insgesamt nicht mehr als 0,1 Gew.-% der Elemente Terbium, Holmium, Erbium, Thulium, Ytterbium und Lutetium enthält.

2. Resorbierbarer Stent aus einer Magnesiumlegierung gemäß Anspruch 1 umfassend:

| | | | |
|---|---|---|---|
| 0,1 Gew.-% | - | 2,0 Gew.-% | Zink |

3. Resorbierbarer Stent aus einer Magnesiumlegierung gemäß Anspruch 1 oder 2 umfassend:

| | | | |
|---|---|---|---|
| 0,1 Gew.-% | - | 0,3 Gew.-% | Zirkonium |

4. Resorbierbarer Stent aus einer Magnesiumlegierung gemäß einem der Ansprüche 1 - 3 enthaltend Verunreinigungen.

5. Resorbierbarer Stent aus einer Magnesiumlegierung gemäß einem der Ansprüche 1 - 4 des Weiteren umfassend:

| | | |
|---|---|---|
| 1 ppm | - | 0,3 Gew.-% Verunreinigungen wie z.B. andere Metalle, Metallsalze und Nichtmetalle. |

6. Resorbierbarer Stent gemäß einem der Ansprüche 1 - 5, wobei die Legierung kein Yttrium und kein Gadolinium enthält.

7. Resorbierbarer Stent aus einer Magnesiumlegierung gemäß einem der Ansprüche 1, 2, 4 oder 5, bestehend aus:

| | | | |
|---|---|---|---|
| 80,7 Gew.-% | - | 94,7 Gew.-% | Magnesium |
| 5,0 Gew.-% | - | 15,0 Gew.-% | Dysprosium |
| 0,1 Gew.-% | - | 2,0 Gew.-% | Neodym |
| 0,1 Gew.-% | - | 2,0 Gew.-% | Zink |
| 0,1 Gew.-% | - | 0,3 Gew.-% | Verunreinigungen wie z.B. anderen Metallen, Metallsalzen und Nichtmetallen, |

wobei die Legierung kein Yttrium und kein Gadolinium enthält.

8. Resorbierbarer Stent aus einer Magnesiumlegierung gemäß einem der Ansprüche 1, 3, 4 oder 5, bestehend aus:

| | | | |
|---|---|---|---|
| 82,4 Gew.-% | - | 94,7 Gew.-% | Magnesium |
| 5,0 Gew.-% | - | 15,0 Gew.-% | Dysprosium |
| 0,1 Gew.-% | - | 2.0 Gew.-% | Neodym |
| 0,1 Gew.-% | - | 0,3 Gew.-% | Zirkonium |
| 0,1 Gew.-% | - | 0,3 Gew.-% | Verunreinigungen wie z.B. andere Metallen, Metallsalzen und Nichtmetallen, |

wobei die Legierung kein Yttrium und kein Gadolinium enthält.

9. Resorbierbarer Stent aus einer Magnesiumlegierung gemäß einem der Ansprüche 1 - 5, bestehend aus:

| 80,4 Gew.-% | - | 94,6 Gew.-% | Magnesium |
|---|---|---|---|
| 5,0 Gew.-% | - | 15,0 Gew.-% | Dysprosium |
| 0,1 Gew.-% | - | 2,0 Gew.-% | Neodym und/oder Europium |
| 0,1 Gew.-% | - | 2,0 Gew.-% | Zink |
| 0,1 Gew.-% | - | 0,3 Gew.-% | Zirkonium |
| 0,1 Gew.-% | - | 0,3 Gew.-% | Verunreinigungen wie z.B. andere Metallen, Metallsalzen und Nichtmetallen, |

wobei die Legierung kein Yttrium und kein Gadolinium enthält.

10. Resorbierbarer Stent aus einer Magnesiumlegierung gemäß Anspruch 9, bestehend aus:

| 79,7 Gew.-% | - | 94,6 Gew.-% | Magnesium |
|---|---|---|---|
| 5,0 Gew.-% | - | 15,0 Gew.-% | Dysprosium |
| 0,1 Gew.-% | - | 2,0 Gew.-% | Neodym |
| 0,1 Gew.-% | - | 2,0 Gew.-% | Zink |
| 0,1 Gew.-% | - | 0,3 Gew.-% | Zirkonium |
| 0,1 Gew.-% | - | 1,0 Gew.-% | Verunreinigungen wie z.B. andere Metallen, Metallsalzen und Nichtmetallen, |

wobei die Legierung kein Yttrium und kein Gadolinium enthält.

11. Resorbierbarer Stent gemäß einem der vorherigen Ansprüche, wobei es sich bei dem resorbierbaren Stent um einen Stent für Blutgefäße, Harnwege, Atemwege, Gallenwege oder den Verdauungstrakt handelt.

12. Magnesiumlegierung die bezogen auf das Gesamtgewicht der Legierung folgende Bestandteile enthält:

| 7,0 Gew.-% - 15,0 Gew.-% | | | Dysprosium |
|---|---|---|---|
| 0,5 Gew.-% | - | 1,5 Gew.-% | Neodym und/oder Europium. |
| 0,5 Gew.-% | - | 1,5 Gew.-% | Zink |
| 0,1 Gew.-% | - | 0,5 Gew.-% | Zirkonium |
| Rest bis 100,0 Gew.-% | | | Magnesium, |

wobei die Legierung insgesamt nicht mehr als 0,1 Gew.-% der Elemente Terbium, Holmium, Erbium, Thulium, Ytterbium und Lutetium enthält.

**Claims**

1. Absorbable stent consisting of a magnesium alloy which contains the following components based on the total weight of the alloy:

| 5.0 % by wt.. | - | 25.5 % by wt | dysprosium |
|---|---|---|---|
| 0.01 % by wt.. | - | 5.0 % by wt. | neodymium and/or europium |
| 0.1 % by wt. | - | 3.0 % by wt. | zinc |
| 0.1 % by wt.. | - | 2.0 % by wt. | zirconium |
| balance to | - | 100.0 % by wt. | magnesium, |

wherein the alloy contains in total not more than 0.1 % per wt. of the elements terbium, holmium, erbium, thulium, ytterbium and lutetium.

2. Absorbable stent of a magnesium alloy according to claim 1 comprising:

0.1 % by wt.      -      2.0 % by wt.    zinc

3. Absorbable stent of a magnesium alloy according to claim 1 or 2 comprising:

0.1 % by wt.    -    0.3 % by wt.    zirconium

4. Absorbable stent of a magnesium alloy according to any of the claims 1 - 3 containing impurities.

5. Absorbable stent of a magnesium alloy according to any of the claims 1 - 4 further comprising:

1 ppm    -    0.3 % by wt.    impurities such as for example other metals, metal salts and non-metals.

6. Absorbable stent according to any of the claims 1 - 5, wherein the alloy contains no yttrium and no gadolinium.

7. Absorbable stent of a magnesium alloy according to any of the claims 1, 2, 4, or 5 consisting of:

| | | | |
|---|---|---|---|
| 80.7 % by wt. | - | 94.7 % by wt. | magnesium |
| 5.0 % by wt. | - | 15.0 % by wt. | dysprosium |
| 0.1 % by wt. | - | 2.0 % by wt. | neodymium |
| 0.1 % by wt. | - | 2.0 % by wt. | zinc |
| 0.1 % by wt. | - | 0.3 % by wt. | impurities such as for example other metals, metal salts and non-metals, |

wherein the alloy contains no yttrium and no gadolinium.

8. Absorbable stent of a magnesium alloy according to any of the claims 1, 3, 4, or 5 consisting of:

| | | | |
|---|---|---|---|
| 82.4 % by wt. | - | 94.7 % by wt. | magnesium |
| 5.0 % by wt. | - | 15.0 % by wt. | dysprosium |
| 0.1 % by wt. | - | 2.0 % by wt. | neodymium |
| 0.1 % by wt. | - | 0.3 % by wt. | zirconium |
| 0.1 % by wt. | - | 0.3 % by wt. | impurities such as for example other metals, metal salts and non-metals, |

wherein the alloy contains no yttrium and no gadolinium.

9. Absorbable stent of a magnesium alloy according to any of the claims 1 - 5, consisting of:

| | | | |
|---|---|---|---|
| 80.4 % by wt. | - | 94.6 % by wt. | magnesium |
| 5.0 % by wt. | - | 15.0 % by wt. | dysprosium |
| 0.1 % by wt. | - | 2.0 % by wt. | neodymium and/or europium |
| 0.1 % by wt. | - | 2.0 % by wt. | zinc |
| 0.1 % by wt. | - | 0.3 % by wt. | zirconium |
| 0.1 % by wt. | - | 0.3 % by wt. | impurities such as for example other metals, metal salts and non-metals, |

wherein the alloy contains no yttrium and no gadolinium.

10. Absorbable stent of a magnesium alloy according to claim 9, consisting of:

| | | | |
|---|---|---|---|
| 79.7 % by wt. | - | 94.6 % by wt. | magnesium |
| 5.0 % by wt. | - | 15.0 % by wt. | dysprosium |

(continued)

| 0.1 % by wt. | - | 2.0 % by wt. | neodymium |
|---|---|---|---|
| 0.1 % by wt. | - | 2.0 % by wt. | zinc |
| 0.1 % by wt. | - | 0.3 % by wt. | zirconium |
| 0.1 % by wt. | - | 1.0 % by wt. | impurities such as for example other metals, metal salts and non-metals, |

wherein the alloy contains no yttrium and no gadolinium.

11. Absorbable stent according to any one of the foregoing claims, wherein the absorbable stent is a stent for blood vessels, urinary tracts, respiratory tracts, biliary tracts or the digestive tract.

12. Magnesium alloy which contains the following components based on the total weight of the alloy:

| 7.0 % by wt. | - | 15.0 % by wt. | dysprosium |
|---|---|---|---|
| 0.5 % by wt. | - | 1.5 % by wt. | neodymium and/or europium |
| 0.5 % by wt. | - | 1.5 % by wt. | zinc |
| 0.1 % by wt. | - | 0.5 % by wt. | zirconium |
| balance to | | 100.0 % by wt. | magnesium, |

wherein the alloy contains in total not more than 0.1 % per wt. of the elements terbium, holmium, erbium, thulium, ytterbium and lutetium.

## Revendications

1. Stent résorbable constitué par un alliage de magnésium qui contient les composants suivants par rapport au poids total de l'alliage:

| 5,0 % par poids | - | 25,5 % par poids | dysprosium |
|---|---|---|---|
| 0,01 % par poids | - | 5,0 % par poids | neodymium et/ou europium |
| 0,1 % par poids | - | 3,0 % par poids | zinc |
| 0,1 % par poids | - | 2,0 % par poids | zirconium |
| le reste jusqu'à | | 100,0 % par poids | magnésium |

où l'alliage ne contient pas au total plus de 0,1 % des éléments terbium, holmium, erbium, thulium, ytterbium et lutétium.

2. Stent résorbable à partir d'un alliage de magnésium selon la revendication 1 comprenant:

| 0,1 % par poids | - | 2,0 % par poids | zinc. |
|---|---|---|---|

3. Stent résorbable à partir d'un alliage de magnésium selon la revendication 1 ou 2 comprenant:

| 0,1 % par poids | - | 0,3 % par poids | zirconium. |
|---|---|---|---|

4. Stent résorbable à partir d'un alliage de magnésium selon une quelconque des revendications 1 - 3 comprenant des impuretés.

5. Stent résorbable à partir d'un alliage de magnésium selon une quelconque des revendications 1 - 4 comprenant de plus:

1 ppm - 0,3 % par poids impuretés, comme par exemple d'autres métaux, sels de métaux et non-métaux.

**6.** Stent résorbable à partir d'un alliage de magnésium selon une quelconque des revendications 1 - 5, où l'alliage ne contient pas d'yttrium ou du gadolinium.

**7.** Stent résorbable à partir d'un alliage de magnésium selon une quelconque des revendications 1, 2, 4 ou 5, consistant en:

| | | | |
|---|---|---|---|
| 80,7 % par poids | - | 94,7 % par poids | magnésium |
| 5,0 % par poids | - | 15,0 % par poids | dysprosium |
| 0,1 % par poids | - | 2,0 % par poids | neodymium |
| 0,1 % par poids | - | 2,0 % par poids | zinc |
| 0,1 % par poids | - | 0,3 % par poids | impuretés comme par exemple d'autres métaux, sels de métaux et non-métaux, |

où l'alliage ne contient pas d'yttrium ou du gadolinium.

**8.** Stent résorbable à partir d'un alliage de magnésium selon une quelconque des revendications 1, 3, 4 ou 5, consistant en:

| | | | |
|---|---|---|---|
| 82,4 % par poids | - | 94,7 % par poids | magnésium |
| 5,0 % par poids | - | 15,0 % par poids | dysprosium |
| 0,1 % par poids | - | 2,0 % par poids | neodymium |
| 0,1 % par poids | - | 0,3 % par poids | zirconium |
| 0,1 % par poids | - | 0,3 % par poids | impuretés comme par exemple d'autres métaux, sels de métaux et non-métaux, |

où l'alliage ne contient pas d'yttrium ou du gadolinium.

**9.** Stent résorbable à partir d'un alliage de magnésium selon une quelconque des revendications 1 - 5, consistant en:

| | | | |
|---|---|---|---|
| 80,4 % par poids | - | 94,6 % par poids | magnésium |
| 5,0 % par poids | - | 15,0 % par poids | dysprosium |
| 0,1 % par poids | - | 2,0 % par poids | neodymium et/ou europium |
| 0,1 % par poids | - | 2,0 % par poids | zinc |
| 0,1 % par poids | - | 0,3 % par poids | zirconium |
| 0,1 % par poids | - | 0,3 % par poids | impuretés, comme par exemple d'autres métaux, sels de métaux et non-métaux, |

où l'alliage ne contient pas d'yttrium ou du gadolinium.

**10.** Stent résorbable à partir d'un alliage de magnésium selon la revendication 9, consistant en:

| | | | |
|---|---|---|---|
| 79,7 % par poids | - | 94,6 % par poids | magnésium |

(suite)

| | | | |
|---|---|---|---|
| 5,0 % par poids | - | 15,0 % par poids | dysprosium |
| 0,1 % par poids | - | 2,0 % par poids | neodymium |
| 0,1 % par poids | - | 2,0 % par poids | zinc |
| 0,1 % par poids | - | 0,3 % par poids | zirconium |
| 0,1 % par poids | - | 1,0 % par poids | impuretés, comme par exemple d'autres métaux, sels de métaux et non-métaux, |

où l'alliage ne contient pas d'yttrium ou du gadolinium.

11. Stent résorbable à partir d'un alliage de magnésium selon une des revendications précédentes, où le stent est un stent pour les vaisseaux sanguins, les voies urinaires, les voies respiratoires, les voies biliaires ou la voie digestive.

12. Alliage de magnésium qui contient les composants suivants par rapport au poids total de l'alliage:

| | | | |
|---|---|---|---|
| 7,0 % par poids | - | 15,0 % par poids | dysprosium |
| 0,5 % par poids | - | 1,5 % par poids | neodymium et/ou europium |
| 0,5 % par poids | - | 1,5 % par poids | zinc |
| 0,1 % par poids | - | 0,5 % par poids | zirconium |
| le reste jusqu'à | | 100,0 % par poids | magnésium |

où l'alliage ne contient pas au total plus de 0,1 % des éléments terbium, holmium, erbium, thulium, ytterbium et lutétium.

# Figur 1

# Figur 2

## Figur 3

## Figur 4

## Figur 5

500 µm

Figur 6 A

**Figur 6 B**

**Figur 6 C**

**Figur 6 D**

## Figur 7

## Figur 8

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 1419793 B1
- EP 2169090 A
- EP 2213314 A1
- EP 1842507 A1